# EUROPEAN PATENT SPECIFICATION

(11) **EP 3 919 476 B1**
(45) Date of publication and mention of the grant of the patent: **11.12.2024**
(21) Application number: 20748885.9
(22) Date of filing: 21.01.2020
(51) Int. Cl.: C07D 211/60, C07D 207/16, C07D 205/04, C07D 223/06, C07C 237/00, A61P 23/02, A61K 31/167

(54) **QUATERNARY AMMONIUM SALT COMPOUND, PREPARATION METHOD THEREFOR AND USE THEREOF**
QUARTÄRE AMMONIUMSALZVERBINDUNG, HERSTELLUNGSVERFAHREN DAFÜR UND VERWENDUNG DAVON
COMPOSÉ DE SEL D'AMMONIUM QUATERNAIRE, SON PROCÉDÉ DE PRÉPARATION ET SON UTILISATION

(30) Priority: 01.02.2019 CN 201910102803
(43) Date of publication of application: 08.12.2021
(73) Proprietor: West China Hospital, Sichuan University, Chengdu, Sichuan 610041 (CN)
(72) Inventor: KE, Bowen, Chengdu, Sichuan 610041 (CN); LIU, Jin, Chengdu, Sichuan 610041 (CN); ZHANG, Wensheng, Chengdu, Sichuan 610041 (CN); YANG, Jun, Chengdu, Sichuan 610041 (CN); TANG, Lei, Chengdu, Sichuan 610041 (CN)
(74) Representative: Altmann Stößel Dick Patentanwälte PartG mbB
(86) International application number: PCT/CN2020/073387
(87) International publication number: WO 2020/156359

(56) References cited:
- EP-A1- 3 750 870
- WO-A1-2018/121427
- CN-A- 1 295 558
- CN-A- 103 601 650
- CN-A- 110 156 665

## Description

### Technical field

The present invention belongs to the field of chemical medicine, and specifically relates to a quaternary ammonium salt compound, as well as a preparation method and a use thereof.

### Background technology

Local anesthetics is a class of drugs that can reversibly block the occurrence and transmission of sensory nerve impulses in the administration area. Under the condition that animals or humans are waking and conscious, it can locally and reversibly block the generation and signal conduction of sensory nerve impulse, resulting in the temporary sensory loss in the innervated area, and thereby reversibly causing the loss of local tissue pain. Generally, the effect of local anesthetics is limited to the administration site and disappears rapidly as the drug diffuses from the administration site. Local anesthetics block the generation of action potentials and the conduction of nerve impulses by directly inhibiting the related ion channels in nerve cells and fiber membranes, thereby producing local anesthesia. Currently, the well-known action mechanism for local anesthetics is blocking voltage-gated Na⁺ channels in nerve cell membranes, inhibiting nerve impulses, thereby producing local anesthesia.

The local anesthetics currently used in clinical practice are all hydrophobic compounds without electric charge, and they can easily enter nerve cells through cell membranes by diffusion and permeation, to reach the blocking site of sodium channels, and thereby interrupt the excitability of neurons by blocking sodium channels. In fact, although these local anesthetic molecules can easily enter nerve cells by diffusion to exert their actions, they also easily diffuse rapidly from the drug delivery site by diffusion, thereby escaping from nerve cells and resulting in that the local anesthetic effect cannot be kept for a long time. Even if the dosage is increased, the local anesthesia time can only be prolonged to a certain extent. These local anesthetic drugs cannot realize the ideal effect of long-time local anesthesia. At present, most of the local anesthetics commonly used in clinical have an action time of less than 4 hours. Because traditional local anesthetics last for a short period of time, analgesic pumps have to be used to maintain nerve block. The use of catheters in the spinal canal, nerve roots, and subcutaneous locations has greatly increased medical costs and the incidence of infection.

On the other hand, traditional local anesthetics do not have specific selectivity for nerve blocking. They block a variety of nerve fibers extensively during use, and affect various nerve functions such as sensation, pain, movement, and sympathetic nerves. This pharmacological feature greatly limits the wide application of local anesthetics in clinical practice. For example, early functional exercise and rehabilitation of patients after knee replacement is particularly important, however, there are no drugs that selectively block pain in the current local anesthetics. Most of surgical patients who use local anesthetics experience the motor nerves being blocked, unable to restore motor function, that limits postoperative rehabilitation. The study on local anesthetics urgently needs to introduce new research ideas and develop long-acting local anesthetics that selectively block sensory function, without affecting motor function, to meet clinical requirements.

The chemical structure of traditional local anesthetics generally contains at least one or more non-amide tertiary N atoms. When N is substituted, the corresponding quaternary ammonium compound will be obtained. The molecular structure of the quaternary ammonium compound has a positive charge, and the ability to penetrate the cell membrane is significantly reduced. For example, once the tertiary amine N atom in lidocaine is substituted with ethyl, a quaternary ammonium compound called QX-314 will be obtained. Similar to QX-314, QX-222 is also another quaternary ammonium salt having similar structure. Because the structures of QX-314 and QX-222 have positive charges, they cannot pass through cell membranes under normal conditions, and thus cannot quickly produce local anesthesia. But once it passes through the cell membrane, it can significantly inhibit sodium ion channels in nerve cells, resulting in a lasting local anesthetic effect (Courtney KR.J Pharmacol Exp Ther.1975,195:225-236). Current research has found that QX-314 can easily enter nerve cells through the activation of TRPV1 channel with the assistance of capsaicin (transient receptor potential channel vanilloid subtype 1 agonist, i.e. TRPV1 Agonist), producing a long-time nerve block (Craig R. Ries. Anesthesiology. 2009; 111:122-126)*.* However, the strong irritation of capsaicin makes it difficult to have application prospects.

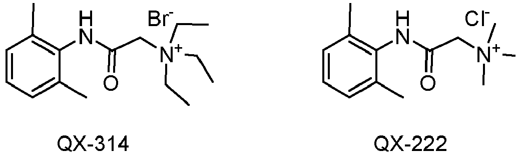

Studies have further shown that the combination of QX314 and local anesthetics clinically used such as bupivacaine and lidocaine can quickly produce anesthesia and avoid the irritation of capsaicin. However, the synergistic use of the above drugs still cannot achieve the expected effect of local anesthesia. With the addition of surfactants, it can also help QX314 enter the cell membrane and cause local anesthesia for more than 8 hours (Daniel S. Kohane, PNAS. 2010;107: 3745-3750)*.* The current research has indicated that QX314 has some safety issues, which are mainly manifested as local nerve damage, and the death of experimental animals during intrathecal injection and so on. Based on QX-314, a series of long-chain compounds with surfactant structure have been developed, and they can realize a longer local anesthetic effect to a certain extent. However, since this kind of compounds have a surfactant-like structure, although they can produce long-acting effect at a certain degree, they will also cause serious muscle and nerve damage in local injection site, with poor safety. Meanwhile, similar compounds that have been reported so far do not have selective local anesthesia, and cannot meet clinical needs. Therefore, whether QX314 is used alone, or it is used in combination with other active drugs, or long-chain compounds of QX314 have structural characteristics of surfactant, have the disadvantages of poor safety and poor selectivity for local anesthesia. Therefore, it is of great significance to study a local anesthetic with fast onset, long-time action, good safety, and specific selectivity. WO 2018/121427, CN 110 156 665 and CN 1 295 558 disclose compounds used as anesthetic drugs.

### Content of the invention

In view of above-mentioned problems, the present invention provides a new class of quaternary ammonium compounds, which have both long-acting and selective local anesthesia (the blocking time of sensory nerve is longer than that of motor nerve), and the compound has the advantages of fast onset, long-time local anesthetic action, good local anesthetic selectivity, less nerve damage, and high safety, compared with the existing QX314, QX314 compositions, and the long-chain compound with surfactant structure characteristics.

The present invention provides compound of formula I, or a pharmaceutically acceptable salt thereof, or a stereoisomer thereof, or a solvate thereof: 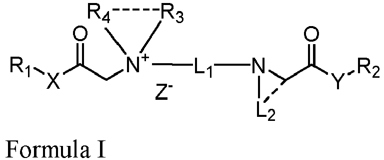 wherein
each of X and Y is independently selected from the group consisting of O and NR₁₀, wherein R₁₀ is selected from the group consisting of H, deuterium, and C₁-C₄ alkyl;
Z⁻ is a pharmaceutically acceptable anion;
R₁ is n₁ R₁₁-substituted aryls;
R₂ is n₁' Rn'-substituted aryls;
wherein n₁ and n₁' are each independently an integer of from 0 to 5, and R₁₁ and R₁₁' are each independently selected from the group consisting of deuterium, C₁-C₄ alkyl, C₁-C₄ alkoxy, halogen, nitro, cyano, hydroxyl, carboxyl, and amino;
when the dotted line between R₃ and R₄ in Formula I is none, R₃ is a substituted C₁-C₁₀ alkyl or unsubstituted C₅-C₁₀ alkyl, and R₄ is independently a substituted or unsubstituted C₁-C₁₀ alkyl, wherein said substituent is selected from the group consisting of deuterium, substituted or unsubstituted C₁-C₄ alkoxy, halogen, nitro, cyano, hydroxyl, carboxy, amino, ester, C₁-C₆ alkylthio, and mercapto; the substituent of said alkoxy is an hydroxyl;
when the dotted line between R₃ and R₄ in formula I is a bond, R₃ and R₄ are independently substituted or unsubstituted C₁-C₄ alkylenyls, and the substituent is an C₁-C₃ alkyl; wherein the main chain of the alkylene contains 0 to 4 heteroatoms, and the heteroatom is selected from the group consisting of O, S, and NR₁₂, wherein said R₁₂ is selected from the group consisting of hydrogen, deuterium, and C₁-C₄ alkyl;
L₁ is a substituted or unsubstituted C₁-C₁₄ alkylenyl; wherein the main chain of the alkylene contains 0 to 4 heteroatoms, and the heteroatom is selected from the group consisting of O, S, and NR₁₂, wherein said R₁₂ is selected from the group consisting of hydrogen, deuterium, C₁-C₄ alkyl, and C₁-C₄ alkoxy; the substituent is selected from the group consisting of deuterium, C₁-C₄ alkyl, C₁-C₄ alkoxy, and halogen;
in formula I, the dotted line of L₂ is a bond, and L₂ is a substituted or unsubstituted C₁-C₈ alkylenyl, and the substituent is selected from the group consisting of deuterium, C₁-C₄ alkyls, C₁-C₄ alkoxys, and halogen.

Further,
each of X and Y is independently NH or NCH₃;
Z⁻ is Br⁻, Cl⁻ or sulfonate;
R₁ is n₁ R₁₁-substituted aryls;
R₂ is n₁' Rn'-substituted aryls;
wherein each of n₁ and n₁' is independently an integer of from 0 to 5, and each of R₁₁ and R₁₁' is independently selected from the group consisting of deuterium, C₁-C₄ alkyl, C₁-C₄ alkoxy, halogen, nitro, cyano, and hydroxyl;
when the dotted line between R₃ and R₄ in Formula I is none, R₃ is a substituted C₁-C₁₀ alkyl or unsubstituted C₅-C₁₀ alkyl, and R₄ is independently a substituted or unsubstituted C₁-C₁₀ alkyl, wherein said substituent is selected from the group consisting of deuterium, substituted or unsubstituted C₁-C₄ alkoxy, hydroxyl, carboxy, C₂-C₅ alkylthio, and mercapto; the substituent of said alkoxy is an hydroxyl;
when the dotted line between R₃ and R₄ in formula I is a bond, R₃ and R₄ are independently C₁-C₄ alkylenyls, wherein the main chain of the alkylene contains 0 to 2 heteroatoms, and the heteroatom is O;
L₁ is a C₃-C₁₄ alkylenyl; wherein the substituent is an alkyl, and the main chain of the alkylene contains 0 to 2 heteroatoms, and the heteroatom is selected from the group consisting of O and S;
in formula I, the dotted line of L₂ is a bond, and L₂ is a C₁-C₆ alkylenyl.

Further,
each of X and Y is independently NH or NCH₃;
Z⁻ is Br⁻, Cl⁻ or sulfonate;
R₁ is n₁ R₁₁-substituted aryls;
R₂ is n₁' Rn'-substituted aryls;
wherein each of n₁ and n₁' is independently an integer of from 2 to 3, and R₁₁ and R₁₁' are independently selected from the group consisting of methyl, propyl, methoxy, hydroxy, nitro, cyano, and halogen;
when the dotted line between R₃ and R₄ in Formula I is none, R₃ is a substituted C₁-C₈ alkyl or unsubstituted C₅-C₁₀ alkyl, and R₄ is independently a substituted or unsubstituted C₁-C₁₀ alkyl, and said substituent is selected from the group consisting of deuterium, substituted or unsubstituted C₁-C₃ alkoxy, hydroxyl, carboxy, C₂-C₅ alkylthio, and mercapto; the substituent of said alkoxy is an hydroxyl;
when the dotted line between R₃ and R₄ in formula I is a bond, R₃ and R₄ are independently substituted or unsubstituted C₂-C₃ alkylenyls, wherein the substituent is a C₁ alkyl, and the main chain of the alkylene contains 0 to 1 heteroatom, and the heteroatom is O;
L₁ is an unsubstituted C₃-C₁₄ alkylenyl; wherein the main chain of the alkylene contains 0 to 2 heteroatoms, and the heteroatom is selected from the group consisting of O and S;
in formula I, the dotted line of L₂ is a bond, and L₂ is an unsubstituted C₁-C₆ alkylenyl.

Further,
each of X and Y is independently NH;
Z⁻ is Br⁻;
R₁ is n₁ R₁₁-substituted aryls;
R₂ is n₁' Rn'-substituted aryls;
wherein each of n₁ and n₁' is independently an integer of from 2 to 3, and R₁₁ and R₁₁' are each a methyl;
when the dotted line between R₃ and R₄ in Formula I is none, R₃ is a substituted C₁-C₈ alkyl or unsubstituted C₅-C₁₀ alkyl; said substituent is selected from the group consisting of deuterium, substituted or unsubstituted C₁-C₃ alkoxy, hydroxyl, carboxy, C₂-C₃ alkylthio, and amercapto; the substituent of said alkoxy is an hydroxyl;
when the dotted line between R₃ and R₄ in formula I is a bond, R₃ and R₄ are independently unsubstituted C₂-C₃ alkylenyls; wherein the main chain of the alkylene contains one heteroatom, and the heteroatom is O;
L₁ is an unsubstituted C₃-C₁₄ alkylenyl; wherein the main chain of the alkylene contains one heteroatom, and the heteroatom is selected from the group consisting of O and S;
in formula I, the dotted line of L₂ is a bond, and L₂ is an unsubstituted C₂-C₆ alkylenyl.

Further, said compound is as shown in formula II: 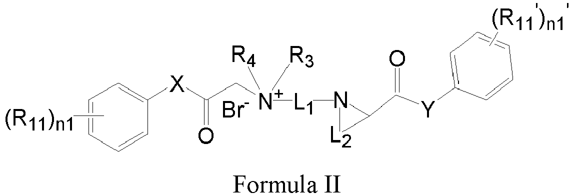 wherein
each of X and Y is independently NH or NCH₃;
each of n₁ and n₁' is independently an integer of from 2 to 3, and R₁₁ and R₁₁' are each independently selected from the group consisting of methyl, propyl, methoxy, hydroxy, nitro, cyano, and halogen;
R₃ is a substituted C₁-C₈ alkyl or unsubstituted C₅-C₁₀ alkyl; R₄ is independently a substituted or unsubstituted C₁-C₁₀ alkyl; the substituent of said alkyl is selected from the group consisting of deuterium, substituted or unsubstituted C₁-C₃ alkoxy, hydroxyl, carboxy, C₂-C₅ alkylthio, and mercapto; the substituent of said alkoxy is an hydroxyl;
L₁ is an unsubstituted C₃-C₁₄ alkylenyl; wherein the main chain of the alkylene contains 0, 1 or 2 heteroatoms, and the heteroatom is selected from the group consisting of O and S;
L₂ is an unsubstituted C₂-C₆ alkylenyl.

Further, said compound is as shown in formula III: 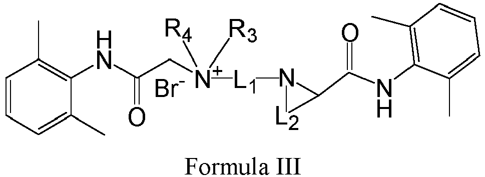 wherein
R₃ is a substituted C₁-C₅ alkyl; R₄ is independently a substituted or unsubstituted C₁-C₅ alkyl; the substituent of said alkyl is an hydroxyl;
L₁ is an unsubstituted C₃-C₆ alkylenyl;
L₂ is an unsubstituted C₄ alkylenyl.

Further, said compound is as shown in formula IV: 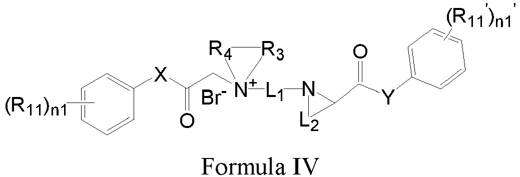 wherein
each of X and Y is NH;
each of n₁ and n₁' is independently an integer of from 2 to 3; R₁₁ and R₁₁' are each a methyl;
R₃ and R₄ are independently substituted or unsubstituted C₂-C₃ alkylenyls; wherein said substituent is a C₁ alkyl, and the main chain of the alkylene contains one heteroatom, and the heteroatom is O;
L₁ is an unsubstituted C₃-C₁₄ alkylenyl; wherein the main chain of the alkylene contains 0 or 1 heteroatom, and the heteroatom is selected from the group consisting of O and S;
L₂ is an unsubstituted C₂-C₆ alkylenyl.

Further, the structure of the compound is one of the followings:

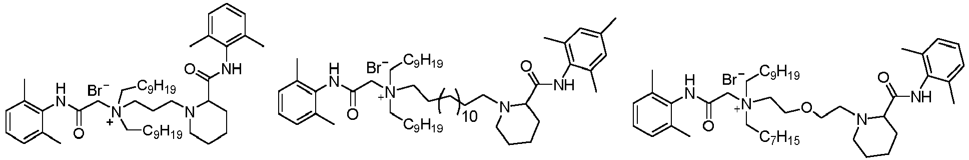

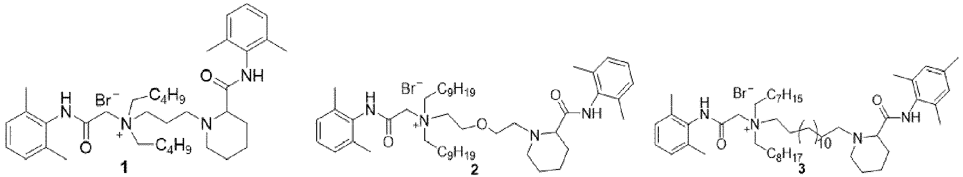

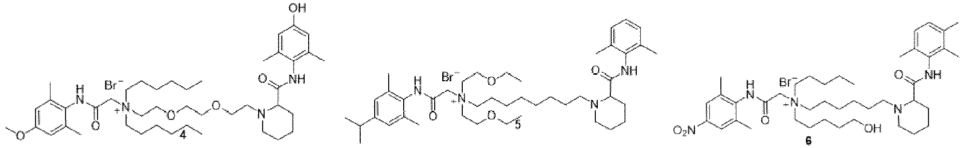

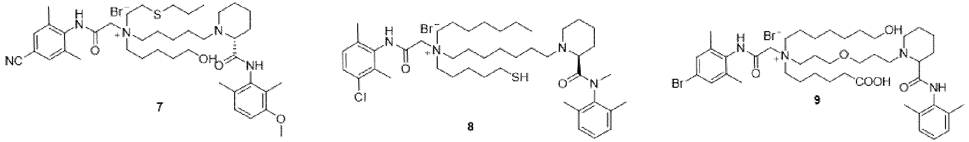

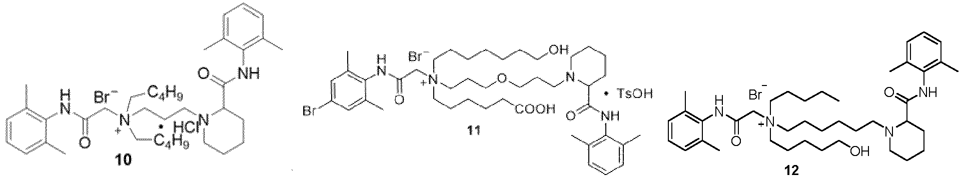

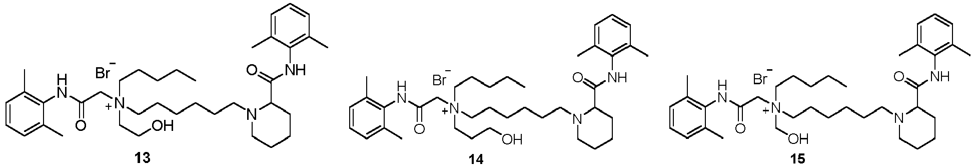

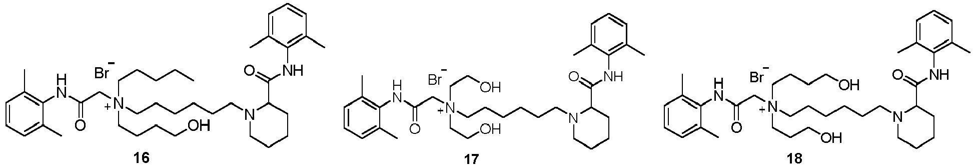

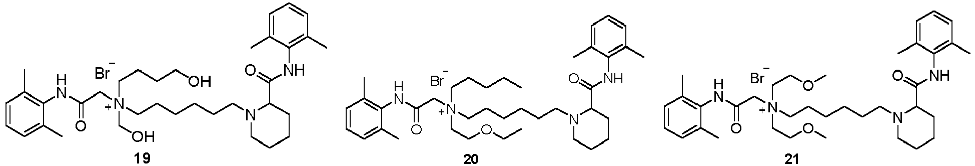

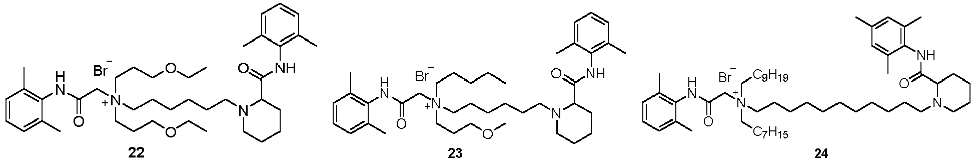

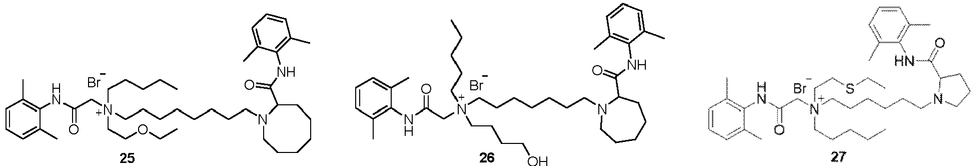

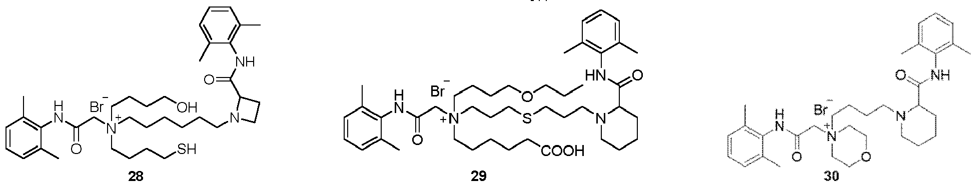

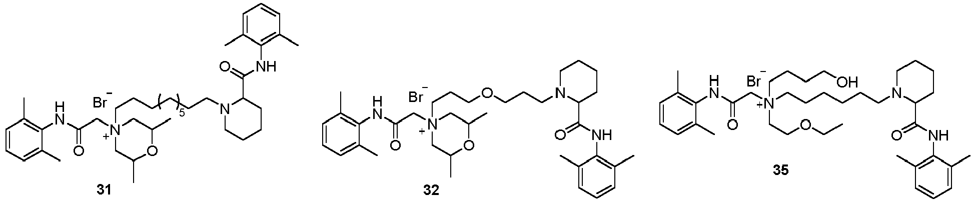

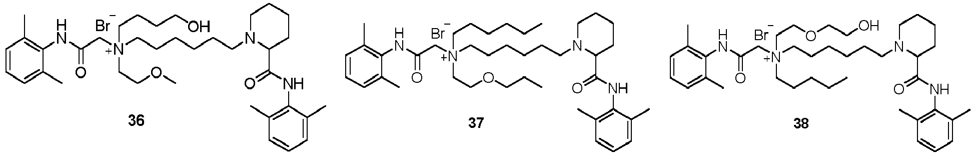

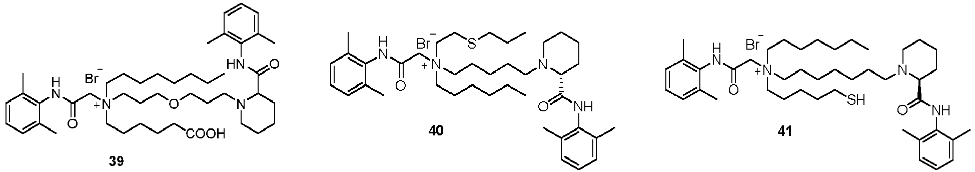

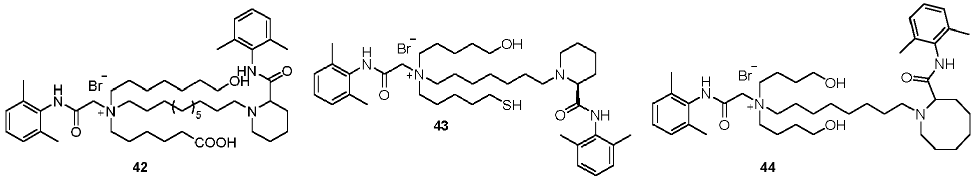

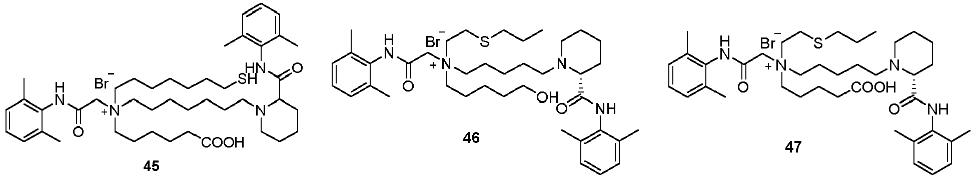

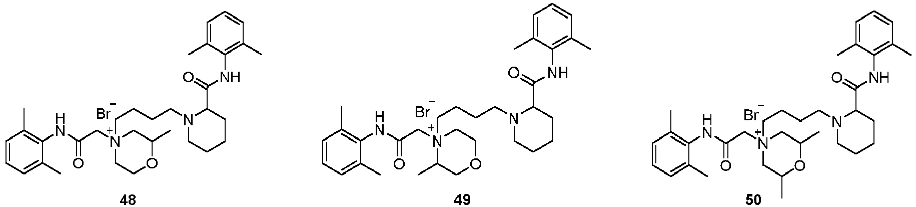

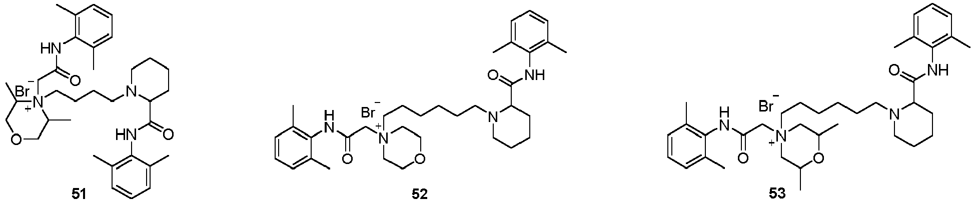

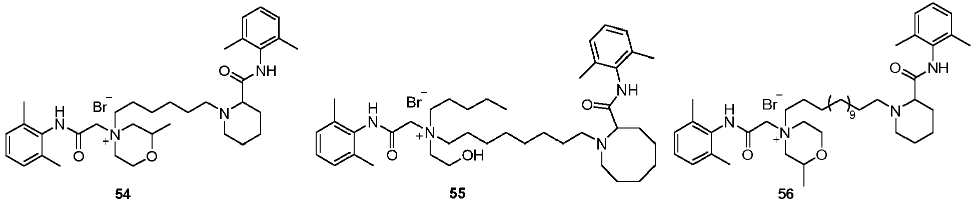

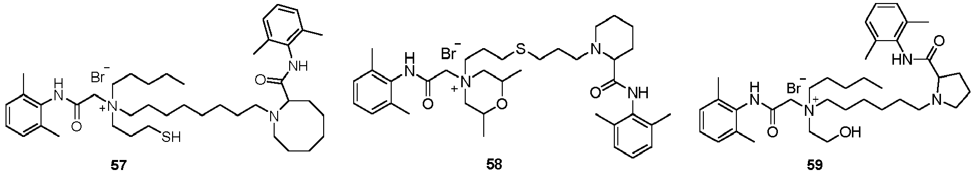

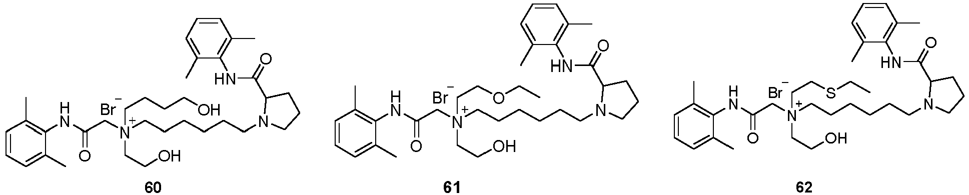

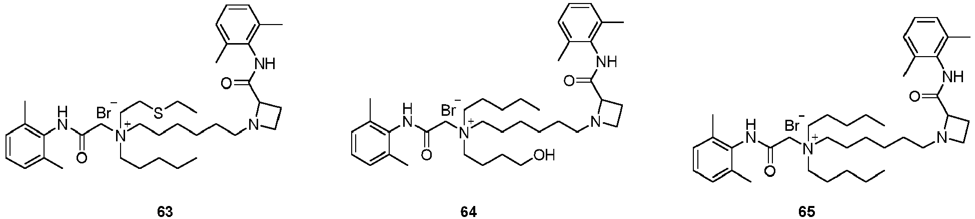

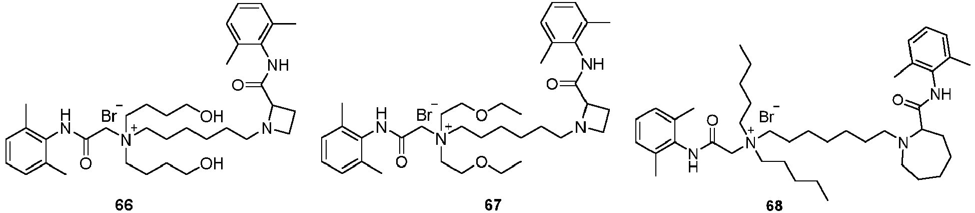

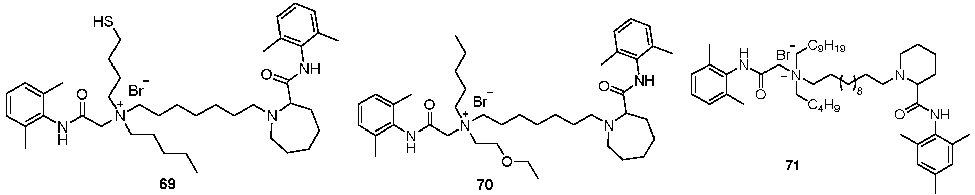

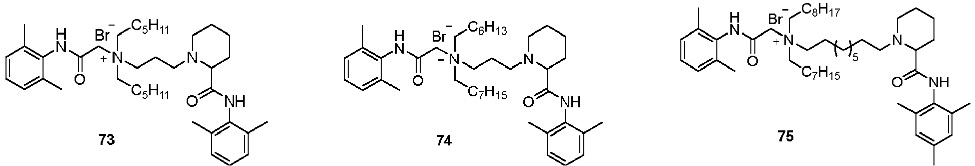

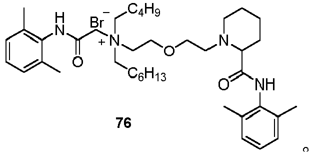

The present invention also provides a use of a composition for preparing a local anesthetic medicine, and said composition is formed by the compound mentioned above, or a pharmaceutically acceptable salt thereof, or a stereoisomer thereof, or a solvate thereof, together with a pharmaceutically acceptable carrier.

Further, said local anesthetic medicine makes the blocking time of sensory nerve longer than that of motor nerve; and/or
said local anesthesia is long-acting local anesthesia;
preferably, the anesthesia time of said local anesthesia exceeds 24 hours.

The present invention also provides a drug, which is a composition formed by the compound mentioned above, or a pharmaceutically acceptable salt thereof, or a stereoisomer thereof, or a solvate thereof, with the addition of pharmaceutically acceptable excipients.

The compounds and derivatives provided in the present invention can be named according to IUPAC (International Union of Pure and Applied Chemistry) or CAS (Chemical Abstracting Service, Columbus, OH) naming system.

For the definition of the terms used in the present invention: unless otherwise specified, the initial definition provided for the group or the term herein is applicable to those in the whole specification; for terms not specifically defined herein, according to the disclosure content and the context, the term should have the meaning commonly given by those skilled in the field.

"Substitution" means that the hydrogen in a molecule is substituted by other different atoms or molecules.

Halogen is fluorine, chlorine, bromine, or iodine.

"Alkyl" is a hydrocarbon group formed by losing one hydrogen in an alkane molecule, such as methyl -CH₃, ethyl -CH₃CH₂, etc. "C₁₋₄ alkyl" denotes a straight or branched hydrocarbon chain containing 1 to 4 carbon atoms.

"Alkylenyl" denotes the hydrocarbon group formed by losing two hydrogens in the alkane molecule, such as methylene -CH₂-, ethylidene -CH₂CH₂-, etc. "C₁₋₄ alkylenyl" denotes a straight or branched hydrocarbon chain containing 1 to 4 carbon atoms.

"Substituted or unsubstituted C₁-C₁₂ alkyl" denote C₁-C₁₂ alkyl that can be substituted or not be substituted.

"L₁ is a substituted or unsubstituted C_{1~12} alkylenyl; wherein, the main chain of said alkylenyl contains 0 to 4 heteroatoms" means a straight or branched hydrocarbon chain containing 1 to 12 carbon atoms; said hydrocarbon chain can be substituted or unsubstituted; "the main chain of said hydrocarbon contains heteroatoms" means that a carbon in the main chain is substituted with a heteroatom, which is O, S, or N substituted.

"Aryl" denotes all-carbon monocyclic or fused polycyclic (i.e. ring sharing adjacent carbon atom pairs) group with conjugated π electron system, such as phenyl and naphthyl. Said aryl ring can be fused to other cyclic groups (including saturated and unsaturated rings), but can not contain heteroatoms such as nitrogen, oxygen, or sulfur. At the same time, the point connecting with the parent must be on the carbon in the ring having the conjugated π electron system. Aryls can be substituted or unsubstituted, i.e. aryls can be substituted by 0 to 4 deuterium, C₁-C₄ alkyl, C₁-C₄ alkoxy, C₁-C₄ alkylthio, halogen, nitro, cyano, hydroxyl, carboxyl, amino, and so on.

The term "pharmaceutically acceptable salt" denotes the salt formed by the compound of the present invention and pharmaceutically acceptable inorganic and organic acids, which is suitable for contacting the tissue of the object (e.g. human) without undue side effects. Among them, the preferred inorganic acids include (but not limited to) hydrochloric acid, hydrobromic acid, phosphoric acid, nitric acid, and sulfuric acid; the preferred organic acids include (but not limited to) formic acid, acetic acid, propionic acid, succinic acid, naphthalene disulfonic acid (1,5), asiatic acid, oxalic acid, tartaric acid, lactic acid, salicylic acid, benzoic acid, valeric acid, diethylacetic acid, malonic acid, succinic acid, fumaric acid, pimelic acid, adipic acid, maleic acid, malic acid, sulfamic acid, phenylpropionic acid, gluconic acid, ascorbic acid, niacin, isoniacin, methanesulfonic acid, p-toluenesulfonic acid, citric acid, and amino acids.

The term "solvate" denotes the solvate formed by the compound of the present invention and pharmaceutically acceptable solvents, in which the pharmaceutically acceptable solvent includes (but not limited to) water, ethanol, methanol, isopropanol, propylene glycol, tetrahydrofuran, and dichloromethane.

The term "pharmaceutically acceptable stereoisomer" means that the chiral carbon atom involved in the compound of the present invention may be R-configuration, S-configuration, or a combination thereof.

The present invention provides a class of quaternary ammonium compounds with novel structures, as well as the preparation method and the use thereof. The compound has a fast onset of action, a long-time local anesthetic (more than 24 hours, and the local anesthesia time for most compounds exceeding 40 hours) effect after a single administration, a selectivity for nerve block (the blocking time of sensory nerve is longer than that of motor nerve, and the difference time is greater than or equal to 5 hours, and the difference time for most compounds is greater than 10 hours), and has both long-acting and selective local anesthetic effect, that significantly reduced the side effects of QX314, QX314 compositions, and the quaternary ammonium compound with surfactant structure characteristics. Moreover, the compound has better safety, that is, the compound of the present invention and its pharmaceutically acceptable salts can be used to prepare safe drugs with long-acting and selective local anesthesia, which has the advantages of long-time local anesthetic action, good local anesthetic selectivity, less nerve damage, and high safety.

By following specific examples of said embodiments, above content of the present invention is further illustrated. But it should not be construed that the scope of the above subject matter of the present invention is limited to the following examples.

### Examples

The starting materials and equipment used in the specific examples of the present invention are all known products and can be obtained by purchasing commercially available items.

### Example 1 Preparation of the compound according to the present invention

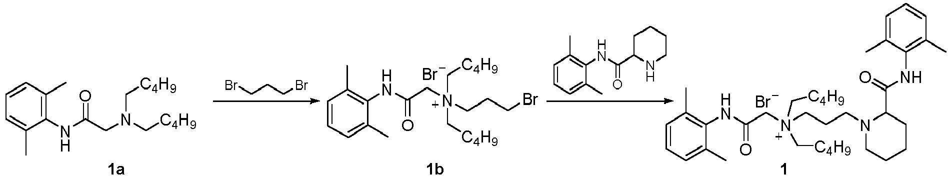

Compound **1a** (5.0 g, 45.39 mmol) was dissolved in 1,3-dibromopropane (15 mL), and the mixture was heated to 75 °C and reacted for 40 h. The reaction was monitored by TLC (DCM: MeOH = 10:1, Rf = 0.3). A suitable amount of ethyl acetate was added to form a viscous syrupy substance. The supernatant was poured out, to obtain the residue of 6 g crude product, which was dissolved in 30 mL methanol and mixed with silica gel. After dry loading, the crude product was purified by silica gel column chromatography, using eluent CH₂Cl₂:MeOH = 10:1. The eluent was collected and concentrated to obtain 3 g of crude product. The resultant product was recrystallized in ethyl acetate and dichloromethane, to prepare 2.5 g of an off-white solid powder (**1b**) with a yield of 31.6%, which was used in the next reaction. Intermediate **1b** (2.00 g, 3.66 mmol) prepared above and N-(2,6-dimethylphenyl)-2-piperidinecarboxamide (0.934 g, 4.03 mmol, CAS: 15883-20-2) were dissolved in 20 mL ethanol, to which was added DIPEA (0.94 g, 1.21 mL, 7.32 mmol). The mixture was warmed to 80 °C and kept for 40 hours. Then, the solvent was evaporated, and the crude product was purified by silica gel column chromatography, using eluent: CH₂Cl₂:MeOH = 10:1. The eluate was collected and concentrated to obtain 1.2 g of a white solid (1). Yield: 48.19%. ¹H NMR (300 MHz, CDCl₃) *δ* (ppm): 10.35 (s, 1 H), 7.84 (s, 1 H), 7.11-7.01 (m, 6 H), 4.89 (s, 2 H), 3.80-3.45 (m, 7 H), 2.71-2.57 (m, 4 H), 2.28-2.17 (m, 12 H), 1.90-1.63 (m, 8 H), 1.60-1.27 (m, 12 H), 1.02-0.87 (m, 6H). HRMS: *m*/*z* 591.9048 [C₃₇H₅₉N₄O₂]⁺.

### Example 2 Preparation of the compound according to the present invention

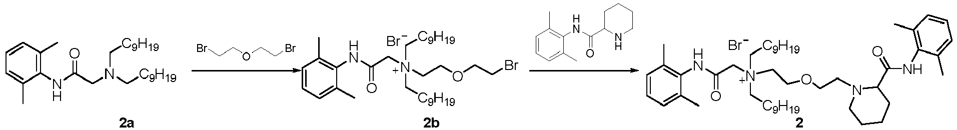

Compound 2a (2.0 g, 40.32 mmol) was dissolved in 2-bromoethyl ether (5 mL), and the mixture was heated to 75 °C and reacted for 24 h. The reaction was monitored by TLC (DCM: MeOH = 10:1, Rf = 0.3). A suitable amount of ethyl acetate was added, then the reaction solution solidified to produce white solids, and 3 g of crude product was filtered out as white solid, that was purified by silica gel column chromatography, using eluent CH₂Cl₂: MeOH = 20:1. The eluent was collected and concentrated to obtain 5.9 g of a white solid (intermediate **2b**), with a yield of 31.5%, which was used in the next reaction.

Intermediate **2b** (1.0 g, 2.16 mmol) prepared above and N-(2,6-dimethylphenyl)-2-piperidinecarboxamide (0.55 g, 2.37 mmol, CAS: 15883-20-2) were dissolved in 15 mL ethanol, to which was added DIPEA (0.53 g, 0.68 ml, 4.12 mmol). The mixture was allowed to react for 10 days at the temperature of 30 °C. Then, the solvent was evaporated, and the crude product was purified by silica gel column chromatography, using eluent CH₂Cl₂:MeOH = 10:1. The eluate was collected and concentrated to obtain 995 mg of a solid as white powder (**2**). Yield: 75.1%. ¹ H NMR (300 MHz, CDCl₃) *δ* (ppm): 9.79 (s, 1 H), 7.60 (s, 1 H), 7.02-6.90 (m, 6 H), 4.33 (s, 2 H), 3.63-3.41 (m, 3 H), 3.25-3.01 (m, 6 H), 2.87-2.68 (m, 6 H), 2.08 (s, 6 H), 2.07 (s, 6 H), 1.89-1.74 (m, 6 H), 1.60-1.40 (m, 4 H), 1.40-1.20 (m, 28 H), 1.05-0.88 (m, 6H). HRMS: *m*/*z* 763.2013 [C₄₈H₈₁N₄O₃]⁺.

### Example 3 Preparation of the compound according to the present invention

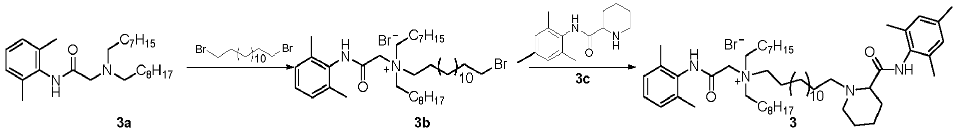

Compound **3a** (500 mg, 1.2 mmol) and 1,14-dibromotetradecane (2 g, 6.0 mmol) were dissolved in acetonitrile (5 mL), and the mixture was heated to 70 °C and reacted for 24 h. The reaction was monitored by TLC (DCM: MeOH = 20:1, Rf = 0.3). A suitable amount of ethyl acetate was added, then the reaction solution solidified to produce white solids, and 0.9 g of crude product was filtered out as white solid, which was purified by silica gel column chromatography, using eluent CH₂Cl₂:MeOH = 20:1. The eluent was collected and concentrated to obtain 500 mg of a white powder solid (**3b**), with a yield of 54.1%, which was used in the next reaction.

Intermediates **3b** (500 mg, 0.65 mmol) and **3c** (0.18 g, 0.71 mmol) prepared above were dissolved in the solvent mixture of 30 mL ethanol and 5 mL methanol, to which was added DIPEA (0.17 g, 0.21 mL, 1.3 mmol). The mixture was allowed to react for 10 days at the temperature of 30 °C. After completion of the reaction, the crude product was purified by silica gel column chromatography, using eluent CH₂Cl₂:MeOH = 10:1. The eluate was collected and concentrated to obtain 400 mg white powder solid (**3**). Yield: 39.6%. ¹H NMR (300 MHz, CDCl₃) *δ* (ppm): 10.24 (s, 1 H), 8.39 (s, 1 H), 7.05-6.87 (m, 5 H), 4.38 (s, 2 H), 3.60-3.50 (m, 4 H), 3.30-3.05 (m, 3 H), 2.45-2.32 (m, 4 H), 2.26 (s, 3 H), 2.17-2.14 (m, 12 H), 2.05-1.83 (m, 8 H), 1.55-1.23(m, 48 H), 1.02-0.83 (m, 6 H). HRMS: *m*/*z* 858.4177 [C₅₆H₉₇N₄O₂]⁺.

### Example 4 Preparation of the compound according to the present invention

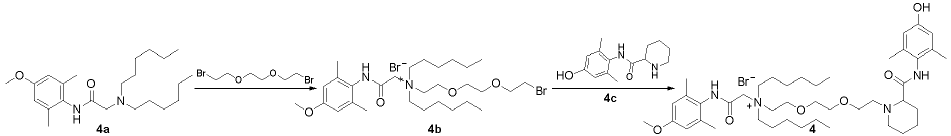

Compound **4a** (1 g, 2.7 mmol) was dissolved in bromo-PEG3-alcohol (3 mL), and the mixture was heated to 75 °C and reacted for 40 h. The reaction was monitored by TLC (DCM: MeOH = 10:1). A suitable amount of ethyl acetate was added to form a viscous syrupy substance. The supernatant was poured out, and the residual solid was dissolved, mixed with silica gel, and purified by silica gel column chromatography, with eluent CH₂Cl₂:MeOH = 10:1. The eluent was collected and concentrated to obtain 1.6 g of crude product. The resultant product was recrystallized in ethyl acetate and dichloromethane, to prepare 1.2 g of an off-white solid powder (**4b**), which was directly used in the next reaction.

Intermediates **4b** (1.00 g, 1.54 mmol) and **4c** (420 mg, 1.7 mmol) prepared above were dissolved in 10 mL ethanol, to which was added DIPEA (0.88 mL, 5.3 mmol). The mixture was heated to 80 °C and kept for 40 hours. Then, the solvent was evaporated, and the crude product was purified by silica gel column chromatography, using eluent: CH₂Cl₂:MeOH = 10:1. The eluate was collected and concentrated to obtain 500 mg of a white solid (**4**). Yield: 39.7%. ¹H NMR (300 MHz, CDCl₃) *δ* (ppm): 10.50 (s, 1 H), 9.19 (s, 1 H), 8.13 (s, 1 H), 7.11-7.01 (m, 4 H), 4.73 (s, 2 H), 3.81-3.73 (m, 6 H), 3.56-3.23 (s, 13 H), 2.53-2.40 (m, 3 H), 2.27 (s, 6 H), 2.23 (s, 6 H), 2.95-1.78 (m, 8 H), 1.42-1.29 (s, 14H), 0.99-0.88 (m, 6 H). HRMS: *m*/*z* 740.0623 [C₄₃H₇₁N₄O₆]⁺.

### Example 5 Preparation of the compound according to the present invention

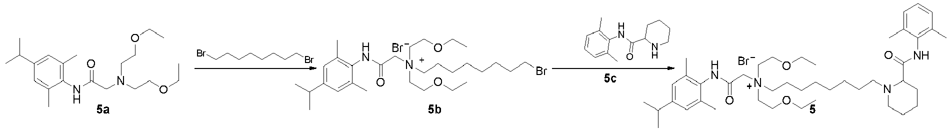

Compound **5a** (5.0 g, 13.7 mmol) was dissolved in 10 mL of 1,8-dibromooctane, and the mixture was heated to 70 °C and reacted. The reaction was monitored by TLC (DCM: MeOH = 10:1). After completion of the reaction, the solvent was evaporated, and the crude product was purified by silica gel column chromatography, using eluent CH₂Cl₂: MeOH = 20: 1. The eluent was collected and concentrated to obtain 5 g of a dark brown compound (**5b**), with a yield of 57.5%, which was used in the next reaction.

Intermediates **5b** (1 g, 1.58 mmol) and **5c** (0.40 g, 1.74 mmol) prepared above were dissolved in 15 mL ethanol, to which was added DIPEA (0.52 g, 3.15 mmol). The mixture was allowed to react for 13 days at the temperature of 30 °C, and the reaction was detected by TLC (DCM : MeOH = 10 : 1). After completion of the reaction, the solvent was evaporated, and the crude product was purified by silica gel column chromatography, using eluent CH₂Cl₂:MeOH = 10:1. The eluate was collected and concentrated to obtain 600 mg of a white solid (**5**). Yield: 48.8%. ¹ H NMR (300 MHz, CDCl₃) *δ* (ppm): 9.81 (s, 1H), 8.69 (s, 1 H), 7.06-6.90 (m, 5 H), 4.64 (s, 2 H), 3.81-3.76 (m, 4 H), 3.41-3.22 (m, 11 H), 3.07-2.95 (m, 1 H), 2.65-2.42 (m, 4 H), 2.15-1.95 (m, 12 H), 1.80-1.60 (m, 10 H), 1.60-1.12 (m, 14 H),1.01-0.88 (m, 6 H). HRMS: *m*/*z* 708.0641 [C₄₃H₇₁N₄O₄]⁺.

### Example 6 Preparation of the compound according to the present invention

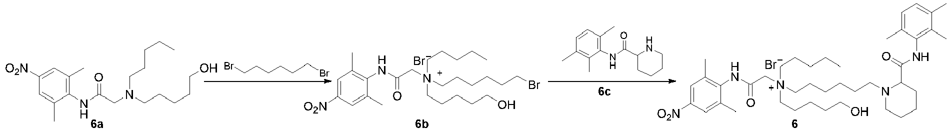

Compound **6a** (2.0 g, 5.28 mmol) was dissolved in 1,6-dibromohexane (8 mL), and the mixture was heated to 75 °C and reacted for 30 h. The reaction was monitored by TLC (DCM: MeOH = 10:1, Rf = 0.3). A suitable amount of ethyl acetate was added to form a viscous syrupy substance. The supernatant was poured out, to obtain the residue of 3 g of crude product, which was dissolved in 30 mL methanol and mixed with silica gel. After dry loading, the crude product was purified by silica gel column chromatography, with eluent CH₂Cl₂: MeOH = 10:1. The eluent was collected and concentrated to obtain 1.5 g of crude product. The resultant product was recrystallized in ethyl acetate and dichloromethane, to prepare 1.2 g of an off-white solid powder (**6b**) with a yield of 36.6%, which was used in the next reaction.

Intermediates **6b** (1.00 g, 1.54 mmol) and **6c** (0.44 g, 1.77 mmol) prepared above were dissolved in 10 mL ethanol, to which was added DIPEA (0.42 g, 0.53 mL, 3.22 mmol). The mixture was heated to 80 °C and kept for 50 hours. Then, the solvent was evaporated, and the crude product was purified by silica gel column chromatography, using eluent: CH₂Cl₂:MeOH = 10:1. The eluate was collected and concentrated to obtain 400 mg of a white solid (**6**). Yield: 31.6%. ¹H NMR (300 MHz, CDCl₃) *δ* (ppm): 10.35 (s, 1 H), 9.89 (s, 1 H), 7.84 (m, 3 H), 7.11-7.01 (m, 2 H), 4.77 (s, 2 H), 3.51-3.45 (m, 3 H), 3.28-3.19 (m, 6 H), 2.71-2.57 (m, 4 H), 2.28-2.07 (m, 15 H), 2.02 (m, 1 H), 1.90-1.63 (m, 12 H), 1.60-1.27 (m, 13 H), 0.95-0.88 (m, 3 H). HRMS: *m*/*z* 709.0084 [C₄₁H₆₆N₅O₅]⁺.

### Example 7 Preparation of the compound according to the present invention

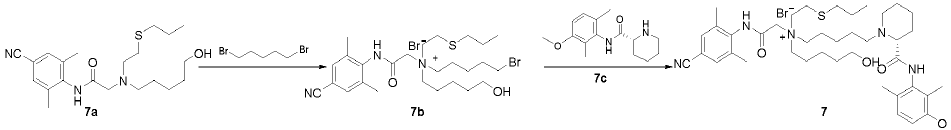

Compound **7a** (2.0 g, 5.11 mmol) was dissolved in 1,5-dibromopentane (10 mL), and the mixture was heated to 75 °C and reacted for 50 h. The reaction was monitored by TLC (DCM: MeOH = 10:1). A suitable amount of ethyl acetate was added to form a viscous syrupy substance. The supernatant was poured out, and the residue was dissolved and mixed with silica gel. After dry loading, the crude product was purified by silica gel column chromatography, with eluent CH₂Cl₂: MeOH = 10:1. The eluent was collected and concentrated to obtain 2.0 g crude product. The crude product was recrystallized in ethyl acetate and dichloromethane, to prepare 1.4 g of an off-white solid powder (**7b**), which was used in the next reaction.

Intermediates **7b** (1.00 g, 1.58 mmol) and **7c** (431 mg, 1.74 mmol) prepared above were dissolved in 10 mL ethanol, to which was added DIPEA (0.52 mL, 3.16 mmol). The mixture was heated to 80 °C and kept for 40 hours. Then, the solvent was evaporated, and the crude product was purified by silica gel column chromatography, using eluent: CH₂Cl₂:MeOH = 10:1. The eluate was collected and concentrated to obtain 450 mg of a white solid (7). Yield: 37.0%. ¹H NMR (300 MHz, CDCl₃) *δ* (ppm): 9.58 (s, 1H), 8.13 (s, 1 H), 7.11-7.01 (m, 4 H), 4.85 (s, 2 H), 3.77 (s, 3 H), 3.65-3.42 (s, 5 H), 3.31-3.24 (m, 4 H), 2.86-2.79 (m, 2 H), 2.43-2.18 (m, 9 H), 2.15-2.08 (m, 9 H), 2.17-1.96 (m, 4 H), 1.76 (s, 8 H), 1.60-1.42 (m, 12 H). HRMS: [C₄₁H₆₄N₅O₄S] ⁺, 723.0535.

### Example 8 Preparation of the compound according to the present invention

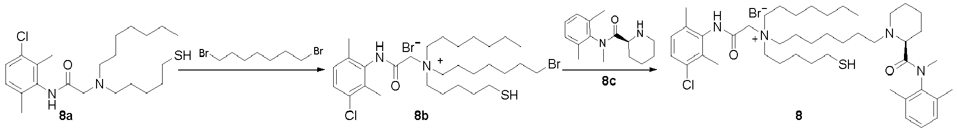

Compound **8a** (2.0 g, 4.85 mmol) was dissolved in 1,7-dibromoheptane (4 mL), and the mixture was heated to 70 °C and reacted for 48 h. The reaction was monitored by TLC (DCM: MeOH = 20:1, R_{f} = 0.3). A suitable amount of ethyl acetate was added, then the reaction solution solidified to produce white solids, and 3.0 g of crude product was filtered out as white solid, which was purified by silica gel column chromatography, using eluent CH₂Cl₂:MeOH = 20:1. The eluent was collected and concentrated to obtain 1.6 g of a white powder solid (**8b**), with a yield of 49.3%, which was used in the next reaction.

Intermediates **8b** (1.5 g, 2.24 mmol) and **8c** (0.61 g, 2.47 mmol) prepared above were dissolved in the solvent mixture of 30 mL ethanol and 5 mL methanol, to which was added DIPEA (0.58 g, 0.74 mL, 4.48 mmol). The mixture was allowed to react for 12 days at the temperature of 30 °C. After completion of the reaction, the crude product was purified by silica gel column chromatography, using eluent CH₂Cl₂:MeOH = 10:1. The eluate was collected and concentrated to obtain 600 mg of a white powder solid (**3**), with a yield of 32.1%. ¹H NMR (300 MHz, CDCl₃) *δ* (ppm): 10.02 (s, 1 H), 9.89 (s, 1 H), 7.23-6.87 (m, 5 H), 4.48 (s, 2 H), 3.60-3.50 (m, 3 H), 3.45-3.33 (m, 6 H), 2.63-2.40 (m, 6 H), 2.17-2.14 (m, 12 H), 1.93-1.75 (m, 11 H), 1.49-1.31 (m, 22 H), 0.92-0.83 (m, 3 H). HRMS: *m*/*z* 756.5955 [C₄₄H₇₂ClN₄O₂S]⁺.

### Example 9 Preparation of the compound according to the present invention

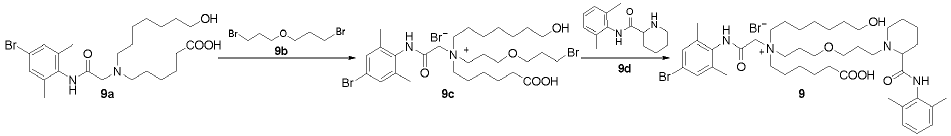

Compounds **9a** (3.0 g, 6.2 mmol) and **9b** (8 g, 31 mmol) was heated to 75 °C and reacted for 40 h. The reaction was monitored by TLC (DCM: MeOH = 10:1). A suitable amount of ethyl acetate was added to form a viscous syrupy substance. The supernatant was poured out, and the residue of the crude product (4.5 g) was dissolved in 30 mL methanol and mixed with silica gel. After dry loading, the crude product was purified by silica gel column chromatography, with eluent CH₂Cl₂: MeOH = 10:1. The eluent was collected and concentrated to obtain 2.5 g of crude product. The crude product was recrystallized in ethyl acetate and dichloromethane, to prepare 2.0 g of an off-white solid powder (**9c**) with a yield of 43.4%, which was used in the next reaction.

Intermediates **9c** (1.00 g, 1.35 mmol) and **9d** (0.344 g, 1.48 mmol) prepared above were dissolved in 10 mL ethanol, to which was added DIPEA (0.35 g, 0.45 mL, 2.70 mmol). The mixture was heated to 80 °C and kept for 40 hours. Then, the solvent was evaporated, and the crude product was purified by silica gel column chromatography, using eluent CH₂Cl₂:MeOH = 10:1. The eluate was collected and concentrated to obtain 400 mg of a white solid (**9**). Yield: 33.2%. ¹H NMR (300 MHz, CDCl₃) *δ* (ppm): 10.05 (s, 1 H), 7.84 (s, 1 H), 7.11- 7.01 (m, 5 H), 4.15 (s, 2 H), 3.60-3.35 (m, 7 H), 3.25-3.18 (s, 8 H), 2.45-2.21 (m, 8 H), 2.14-2.03 (m, 12 H), 1.91-1.56 (m, 12 H), 1.46-1.23 (m, 14 H). HRMS: *m*/*z* 830.9697 [C₄₄H₇₀BrN₄O₆]⁺.

### Example 10 Preparation of the compound according to the present invention

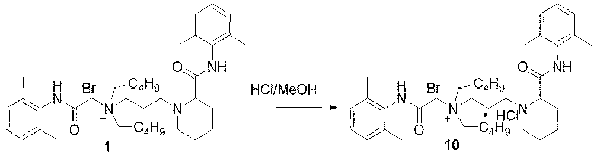

200 mg of the product obtained in Example 1 was dissolved in 10 mL of dichloromethane, to which was added dropwise the solution of 0.1 mol/L hydrochloric acid-methanol at equal molar concention in an ice bath, and then the resultant solution was concentrated to dryness under reduced pressure. The residue was dried in vacuum to provide a pale yellow solid (**10**), with a yield of 97.5%. ¹H NMR (300 MHz, CDCl₃) *δ* (ppm): 10.33 (s, 1 H), 8.68 (s, 1 H), 7.19-7.11 (m, 6 H), 4.93 (s, 2 H), 3.76-3.38 (m, 7 H), 2.70-2.59 (m, 4 H), 2.27-2.18 (m, 12 H), 1.91-1.61 (m, 8 H), 1.64-1.27 (m, 12 H), 1.10-0.88 (m, 6 H). HRMS: *m*/*z* 591.9047 [C₃₇H₅₉N₄O₂]⁺.

### Example 11 Preparation of the compound according to the present invention

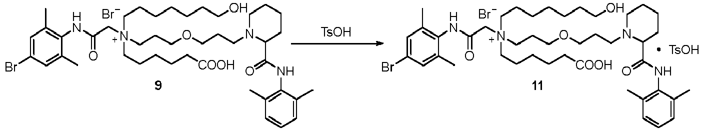

200 mg of the product obtained in Example 9 was dissolved in 10 mL of dichloromethane, to which was added 1 eq p-toluenesulfonic acid, and then the resultant solution was concentrated to dryness under reduced pressure. The residue was dried in vacuum to provide a pale yellow solid (**11**), with a yield of 92.5%.

¹H NMR (300 MHz, CDCl₃) *δ* (ppm): 10.03 (s, 1 H), 8.92 (s, 1 H), 7.13-7.02 (m, 5 H), 4.19 (s, 2 H), 3.62-3.37 (m, 7 H), 3.27-3.19 (s, 8 H), 2.48-2.23 (m, 8 H), 2.17-2.06 (m, 12 H), 1.94-1.58 (m, 12 H), 1.46-1.24 (m, 14 H). HRMS: *m*/*z* 830.9699 [C₄₄H₇₀BrN₄O₆]⁺.

### Example 12 Preparation of the compound according to the present invention

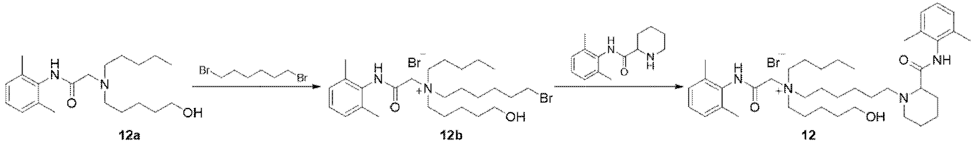

With reference to the method in Example 6, an off-white solid powder was obtained, with a yield of 35.2%.

¹H NMR (300 MHz, CDCl₃) *δ* (ppm): 10.30 (s, 1 H), 9.89 (s, 1H), 7.33 (m, 6 H), 4.72 (s, 1 H), 4.25 (s, 2 H), 3.67 (t, 2 H), 3.45 (t, 1 H), 3.26-3.18 (m, 6 H), 2.51-2.37 (m, 4 H), 2.13 (s, 12 H), 2.02 (m, 2 H), 1.90-1.63 (m, 6 H), 1.60-1.27 (m, 18 H), 0.95-0.88 (m, 3 H). HRMS: *m*/*z* 649.9843 [C₄₀H₆₅N₄O₃]⁺.

### Example 13 Preparation of the compound according to the present invention

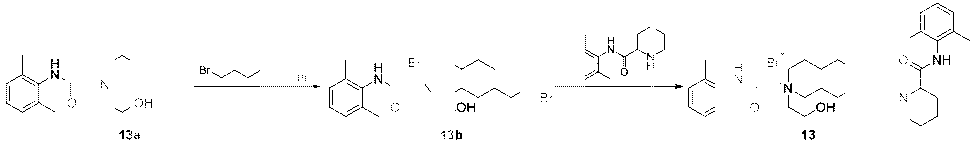

With reference to the method in Example 6, an off-white solid powder was obtained, with a yield of 37.0%.

¹H NMR (300 MHz, CDCl₃) *δ* (ppm): 10.28 (s, 1 H), 9.99 (s, 1H), 7.30 (m, 6 H), 4.31 (s, 1 H), 4.24 (s, 2 H), 3.95 (m, 2 H), 3.40-3.46 (m, 3 H), 3.24-3.16 (m, 4 H), 2.47-2.41 (m, 4 H), 2.12 (s, 12 H), 1.90-1.63 (m, 6 H), 1.57-1.28 (m, 14 H), 0.88 (t, 3 H). HRMS: *m*/*z* 607.9031 [C₃₇H₅₉N₄O₃]⁺.

### Example 14 Preparation of the compound according to the present invention

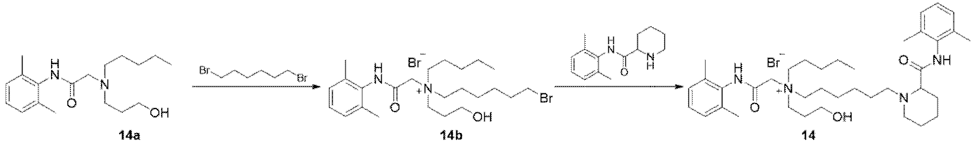

With reference to the method in Example 6, an off-white solid powder was obtained, with a yield of 38.9%.

¹H NMR (300 MHz, CDCl₃) δ (ppm): 10.07 (s, 1 H), 9.73 (s, 1H), 7.11 (m, 6 H), 4.38 (s, 1 H), 4.18 (s, 2 H), 3.42-3.52 (m, 3 H), 3.26-3.14 (m, 6 H), 2.47-2.41 (m, 4 H), 2.15 (s, 12 H), 1.93-1.68 (m, 8 H), 1.55-1.27 (m, 14 H), 0.86 (t, 3 H). HRMS: *m*/*z* 621.9341 [C₃₈H₆₁N₄O₃]⁺.

### Example 15 Preparation of the compound according to the present invention

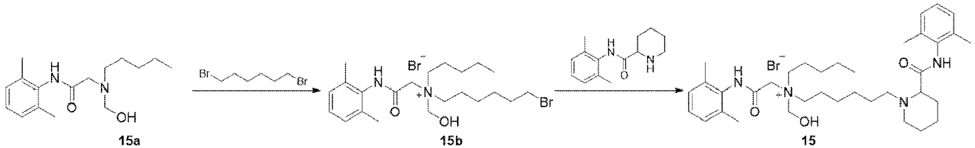

With reference to the method in Example 6, an off-white solid powder was obtained, with a yield of 33.2%.

¹H NMR (300 MHz, CDCl₃) *δ* (ppm): 10.28 (s, 1 H), 9.98 (s, 1H), 7.32 (m, 6 H), 5.47 (s, 2 H), 4.23 (s, 2 H), 4.12 (s, 1 H), 3.40-3.44 (t, 1 H), 3.24-3.17 (m, 4 H), 2.43-2.38 (m, 4 H), 2.12 (s, 12 H), 1.90-1.63 (m, 6 H), 1.57-1.28 (m, 14 H), 0.88 (t, 3 H). HRMS: *m*/*z* 593.8762 [C₃₆H₅₇N₄O₃]⁺.

### Example 16 Preparation of the compound according to the present invention

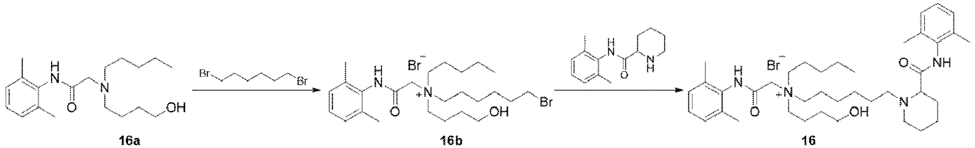

With reference to the method in Example 6, an off-white solid powder was obtained, with a yield of 40.2%.

¹H NMR (300 MHz, CDCl₃) *δ* (ppm): 10.08 (s, 1 H), 9.89 (s, 1H), 7.06 (m, 6 H), 4.18 (s, 2 H), 4.12 (s, 1 H), 3.52-3.45 (m, 3 H), 3.26-3.18 (m, 6 H), 2.53-2.39 (m, 4 H), 2.16 (s, 12 H), 2.05 (m, 2 H), 1.90-1.72 (m, 6 H), 1.61-1.28 (m, 16 H), 0.92-0.87 (m, 3 H). HRMS: *m*/*z* 635.9577 [C₃₉H₆₃N₄O₃]⁺.

### Example 17 Preparation of the compound according to the present invention

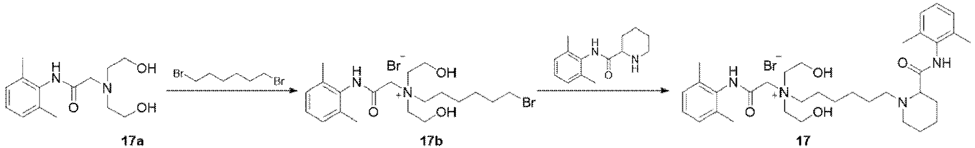

With reference to the method in Example 6, an off-white solid powder was obtained, with a yield of 36.1%.

¹H NMR (300 MHz, CDCl₃) *δ* (ppm): 10.11 (s, 1 H), 9.88 (s, 1H), 7.05 (m, 6 H), 4.28 (s, 2 H), 4.15 (s, 2 H), 3.97-3.84 (m, 4 H), 3.51-3.42 (m, 5 H), 3.26-3.11 (m, 2 H), 2.47-2.32 (m, 4 H), 2.16 (s, 12 H), 1.92-1.72 (m, 4 H), 1.63-1.28 (m, 10 H). HRMS: *m*/*z* 581.8216 [C₃₄H₅₃N₄O₄]⁺.

### Example 18 Preparation of the compound according to the present invention

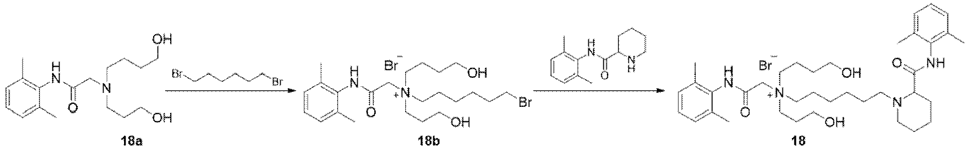

With reference to the method in Example 6, an off-white solid powder was obtained, with a yield of 36.1%.

¹H NMR (300 MHz, CDCl₃) *δ* (ppm): 9.96 (s, 1 H), 9.72 (s, 1H), 7.02 (m, 6 H), 4.28 (s, 1 H), 4.18 (m, 2 H), 4.14 (s, 1 H), 3.51-3.42 (m, 5 H), 3.21-3.11 (m, 6 H), 2.47-2.30 (m, 4 H), 2.15 (s, 12 H), 1.97-1.71 (m, 8 H), 1.66-1.26 (m, 12 H). HRMS: *m*/*z* 623.9029 [C₃₇H₅₉N₄O₄]⁺.

### Example 19 Preparation of the compound according to the present invention

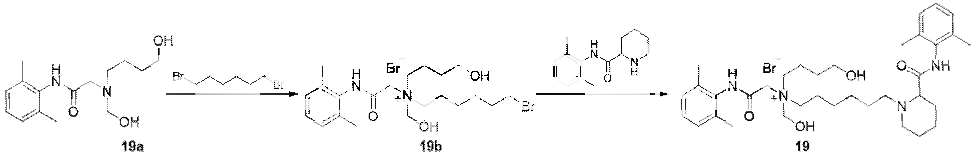

With reference to the method in Example 6, an off-white solid powder was obtained, with a yield of 42.2%.

¹H NMR (300 MHz, CDCl₃) *δ* (ppm): 10.02 (s, 1 H), 9.87 (s, 1H), 7.03 (m, 6 H), 5.48 (s, 2 H), 4.18 (s, 2 H), 4.15 (s, 2 H), 3.52-3.45 (m, 3 H), 3.26-3.18 (m, 4 H), 2.47-2.39(m, 4 H), 2.16 (s, 12 H), 1.92-1.73 (m, 6 H), 1.57-1.28 (m, 12 H). HRMS: *m*/*z* 675.7533 [C₃₅H₅₅N₄O₄]⁺.

### Example 20 Preparation of the compound according to the present invention

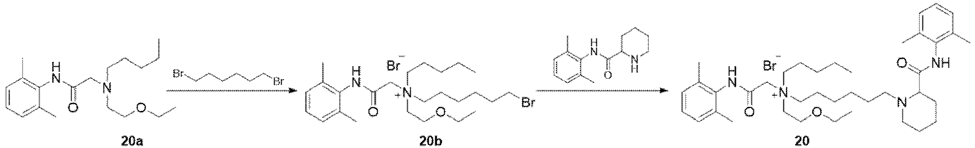

With reference to the method in Example 5, an off-white solid powder was obtained, with a yield of 33.6%.

¹ H NMR (300 MHz, CDCl₃) *δ* (ppm): 10.01 (s, 1 H), 9.88 (s, 1 H), 7.08-6.98 (m, 6 H), 4.25 (s, 2 H), 3.81 (t, 2 H), 3.51-3.28 (m, 5 H), 3.21-2.99 (m, 4 H), 2.52-2.41 (m, 4 H), 2.13-1.98 (m, 12 H), 1.85-1.65 (m, 6 H), 1.53-1.14 (m, 14 H), 1.04 (t, 3 H), 0.87 (t, 3 H). HRMS: *m*/*z* 635.9579 [C₃₉H₆₃N₄O₃]⁺.

### Example 21 Preparation of the compound according to the present invention

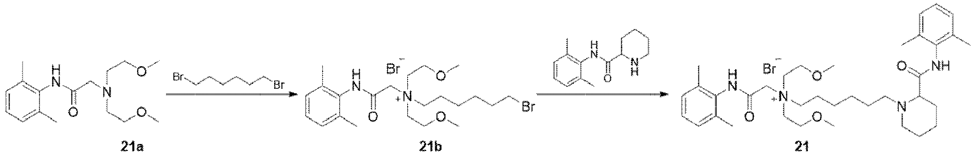

With reference to the method in Example 5, a white solid powder was obtained, with a yield of 28.5%.

¹H NMR (300 MHz, CDCl₃) *δ* (ppm): 10.08 (s, 1 H), 9.75 (s, 1 H), 7.06-6.93 (m, 6 H), 4.53 (s, 2 H), 3.84-3.78 (m, 4 H), 3.42-3.28 (m, 5 H), 3.05-2.96 (m, 8 H), 2.72-2.49 (m, 4 H), 2.16-1.90 (m, 12 H), 1.80-1.63 (m, 4 H), 1.53-1.12 (m, 10 H). HRMS: *m*/*z* 689.7804 [C₃₆H₅₇N₄O₄]⁺.

### Example 22 Preparation of the compound according to the present invention

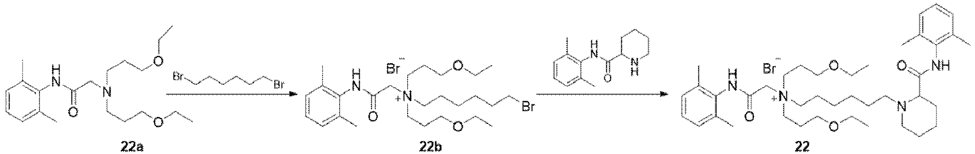

With reference to the method in Example 5, a white solid powder was obtained, with a yield of 30.2%.

¹ H NMR (300 MHz, CDCl₃) *δ* (ppm): 9.92 (s, 1H), 8.76 (s, 1 H), 7.06-6.90 (m, 6 H), 4.53 (s, 2 H), 3.41-3.22 (m, 9 H), 3.07-2.95 (m, 6 H), 2.66-2.42 (m, 4 H), 2.16-1.92 (m, 12 H), 1.80-1.63 (m, 8 H), 1.53-1.12 (m, 10 H), 1.05-0.89 (m, 6 H). HRMS: *m*/*z* 665.9831 [C₄₀H₆₅N₄O₄]⁺.

### Example 23 Preparation of the compound according to the present invention

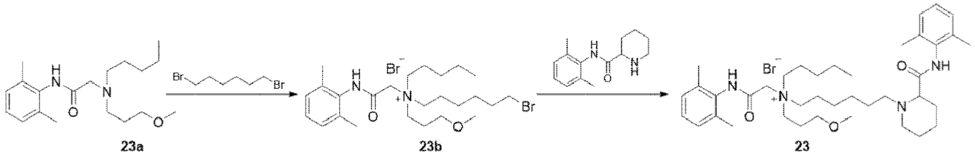

With reference to the method in Example 5, an off-white solid powder was obtained, with a yield of 35.2%.

¹ H NMR (300 MHz, CDCl₃) *δ* (ppm): 10.02 (s, 1 H), 9.65 (s, 1 H), 7.08-6.98 (m, 6 H), 4.25 (s, 2 H), 3.50-3.33 (m, 3 H), 3.26-3,08 (m, 9 H), 2.48-2.36 (m, 4 H), 2.16-2.05 (m, 12 H), 1.85-1.65 (m, 8 H), 1.52-1.17 (m, 14 H), 0.88 (t, 3 H). HRMS: *m*/*z* 635.9571 [C₃₉H₆₃N₄O₃]⁺.

### Example 24 Preparation of the compound according to the present invention

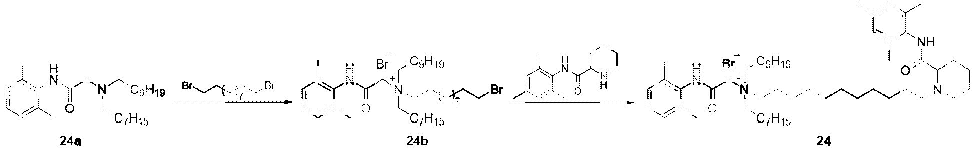

With reference to the method in Example 3, a white solid powder was obtained, with a yield of 43.2%.

¹H NMR (300 MHz, CDCl₃) *δ* (ppm): 10.04 (s, 1 H), 9.39 (s, 1 H), 7.05-6.88 (m, 5 H), 4.38 (s, 2 H), 3.65-3.54 (m, 4 H), 3.33-3.09 (m, 3 H), 2.45-2.32 (m, 4 H), 2.29 (s, 3 H), 2.18-2.15 (m, 12 H), 2.05-1.83 (m, 8 H), 1.48-1.20 (m, 44 H), 1.01-0.83 (m, 6 H). HRMS: *m*/*z* 830.3031 [C₅₄H₉₃N₄O₂]⁺.

### Example 25 Preparation of the compound according to the present invention

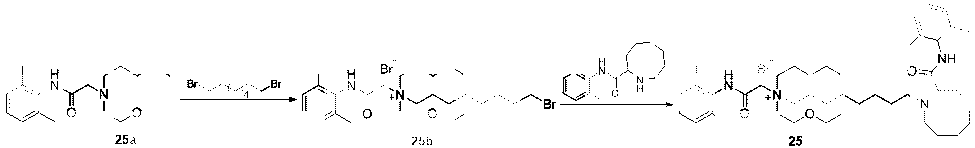

With reference to the method in Example 5, an off-white solid powder was obtained, with a yield of 30.1%.

¹ H NMR (300 MHz, CDCl₃) *δ* (ppm): 10.01 (s, 1 H), 9.88 (s, 1 H), 7.08-6.98 (m, 6 H), 4.25 (s, 2 H), 3.81 (t, 2 H), 3.65 (t, 1 H), 3.51-3.42 (m, 4 H), 3.31-3.13 (m, 4 H), 2.52-2.21 (m, 4 H), 2.14-2.02 (m, 12 H), 1.85-1.49 (m, 6 H), 1.43-1.14 (m, 22 H), 1.05 (t, 3 H), 0.88 (t, 3 H). HRMS: *m*/*z* 692.0656 [C₄₃H₇₁N₄O₃]⁺.

### Example 26 Preparation of the compound according to the present invention

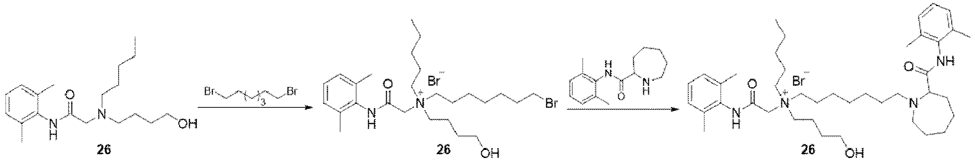

With reference to the method in Example 6, an off-white solid powder was obtained, with a yield of 38.2%.

¹H NMR (300 MHz, CDCl₃) *δ* (ppm): 9.75 (s, 1 H), 7.98 (s, 1H), 7.05 (m, 6 H), 4.21 (s, 2 H), 4.12 (s, 1 H), 3.63 (t, 1 H), 3.52-3.45 (m, 2 H), 3.26-3.18 (m, 6 H), 2.53-2.39 (m, 4 H), 2.16 (s, 12 H), 1.90-1.72 (m, 10 H), 1.61-1.28 (m, 18 H), 0.92-0.87 (m, 3 H). HRMS: *m*/*z* 664.0112 [C₄₁H₆₇N₄O₃]⁺.

### Example 27 Preparation of the compound according to the present invention

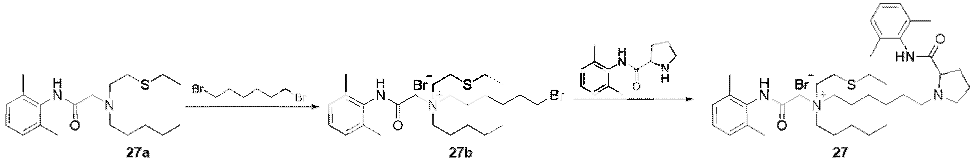

With reference to the method in Example 7, an off-white solid powder was obtained, with a yield of 35.8%.

¹H NMR (300 MHz, CDCl₃) *δ* (ppm): 9.76 (s, 1 H), 8.29 (s, 1 H), 7.18-7.01 (m, 6 H), 4.85 (s, 2 H), 3.61 (t, 2 H), 3.45 (t, 1 H), 3.31-3.24 (m, 4 H), 2.86 (t, 2 H), 2.43-2.18 (m, 6 H), 2.15-2.10 (m, 12 H), 2.05-1.76 (m, 8 H), 1.60-1.22 (m, 10 H), 1.15 (t, 3 H), 0.86 (t, 3 H). HRMS: *m*/*z* 637.9918 [C₃₈H₆₁N₄O₂S]⁺.

### Example 28 Preparation of the compound according to the present invention

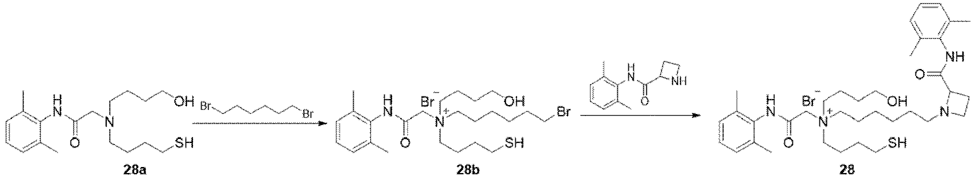

With reference to the method in Example 8, an off-white solid powder was obtained, with a yield of 35.8%.

¹H NMR (300 MHz, CDCl₃) *δ* (ppm): 10.02 (s, 1 H), 9.89 (s, 1 H), 7.23-6.87 (m, 6 H), 4.33-4.14 (m, 4 H), 3.60-3.49 (m, 4 H), 3.33-3.28 (m, 6 H), 2.63-2.39 (m, 6 H), 2.15-2.11 (m, 12 H), 1.93-1.75 (m, 6 H), 1.49-1.31 (m, 4 H), 1.30-1.15 (m, 7 H). HRMS: [C₃₆H₅₇ClN₄O₃S] ⁺, 625.9327.

### Example 29 Preparation of the compound according to the present invention

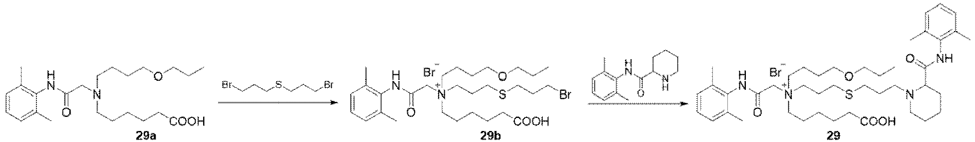

With reference to the method in Example 2, an off-white solid powder was obtained, with a yield of 35.8%.

¹ H NMR (300 MHz, CDCl₃) δ (ppm): 11.88 (s, 1 H), 9.79 (s, 1 H), 8.78 (s, 1 H), 7.09-6.98 (m, 6 H), 4.25 (s, 2 H), 3.52-3.40 (m, 5 H), 3.27-3.21 (m, 4 H), 2.54-2.36 (m, 8 H), 2.22-2.07 (s, 16 H), 1.89-1.74 (m, 6 H), 1.60-1.40 (m, 10 H), 1.40-1.20 (m, 6 H), 0.98 (t, 3 H). HRMS: *m*/*z* 754.1078 [C₄₃H₆₉N₄O₅]⁺.

### Example 30 Preparation of the compound according to the present invention

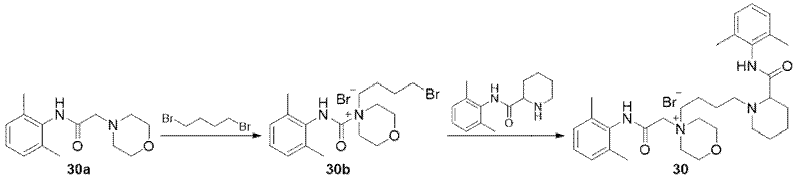

With reference to the method in Example 1, an off-white solid powder was obtained, with a yield of 36.3%.

¹H NMR (300 MHz, CDCl₃) *δ* (ppm): 10.35 (s, 1 H), 9.70 (s, 1 H), 7.11-7.02 (m, 6 H), 4.26 (s, 2 H), 3.88-3.67 (m, 4 H), 3.68-3.45 (m, 5 H), 2.71-2.57 (m, 6 H), 2.28-2.17 (m, 12 H), 1.90-1.63 (m, 4 H), 1.60-1.27 (m, 6 H). HRMS: *m*/*z* 535.7521 [C₃₂H₄₇N₄O₃]⁺.

### Example 31 Preparation of the compound according to the present invention

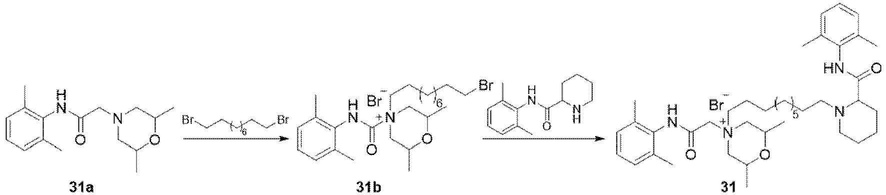

With reference to the method in Example 1, an off-white solid powder was obtained, with a yield of 34.2%.

¹H NMR (300 MHz, CDCl₃) *δ* (ppm): 9.72 (s, 1 H), 8.60 (s, 1 H), 7.07-7.02 (m, 6 H), 4.18 (s, 2 H), 4.02 (m, 2 H), 3.54-3.33 (m, 7 H), 2.52-2.38 (m, 4 H), 2.18-2.06 (m, 12 H), 1.90-1.63 (m, 4 H), 1.60-1.27 (m, 18 H), 1.12-1.03 (m, 6 H). HRMS: *m*/*z* 647.9683 [C₄₀H₆₃N₄O₃]⁺.

### Example 32 Preparation of the compound according to the present invention

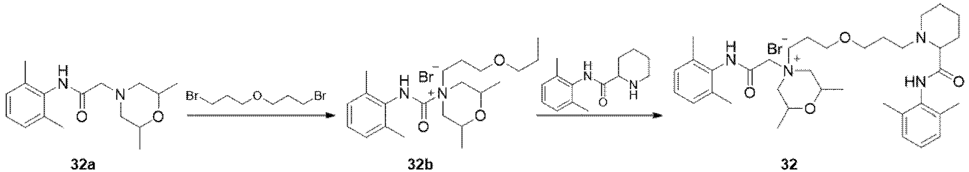

With reference to the method in Example 2, an off-white solid powder was obtained, with a yield of 28.5%.

¹H NMR (300 MHz, CDCl₃) *δ* (ppm): 10.02 (s, 1 H), 9.35 (s, 1 H), 7.11-7.01 (m, 6 H), 4.20 (s, 2 H), 4.03 (m, 2 H), 3.51-3.35 (m, 11 H), 2.45- 2.39 (m, 4 H), 2.15-2.11 (m, 12 H), 1.99-1.72 (m, 4 H), 1.57-1.41 (m, 6 H), 1.18-1.13 (m, 6 H). HRMS: *m*/*z* 607.8599 [C₃₆H₅₅N₄O₄]⁺.

### Example 33 Preparation of the compound according to the present invention

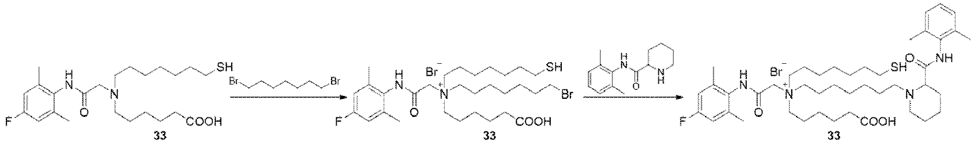

With reference to the method in Example 3, an off-white solid powder was obtained, with a yield of 39.1%.

¹H NMR (300 MHz, CDCl₃) *δ* (ppm): 11.87 (s, 1 H), 10.02 (s, 1 H), 9.69 (s, 1 H), 7.04 (s, 3 H), 6.91-6.85 (m, 2 H), 4.27 (s, 2 H), 3.51-3.29 (m, 7 H), 2.66-2.45 (m, 6 H), 2.31-2.18 (m, 2 H), 2.12 (d, 12 H), 1.98-1.63 (m, 12 H), 1.60-1.27 (m, 21 H). HRMS: *m*/*z* 770.1255 [C₄₄H₇₀FN₄O₄S] ⁺.

### Example 34 Preparation of the compound according to the present invention

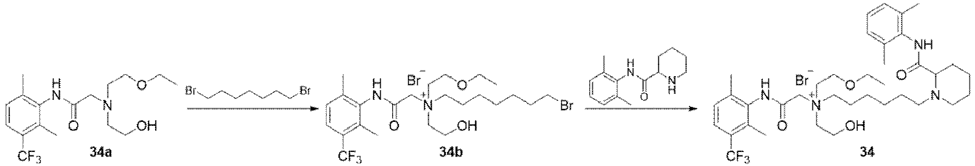

With reference to the method in Example 5, an off-white solid powder was obtained, with a yield of 33.4%.

¹H NMR (300 MHz, CDCl₃) *δ* (ppm): 10.06 (s, 1 H), 9.77 (s, 1 H), 7.18- 7.01 (m, 3 H), 6.85-6.74 (m, 2 H), 4.24 (s, 1 H), 3.97 (t, 2 H), 3.81 (t, 2 H), 3.58-3.16 (m, 11 H), 2.43-2.18 (m, 4 H), 2.15-2.10 (m, 12 H), 2.05-1.76 (m, 4 H), 1.61-1.29 (m, 10 H), 1.05 (t, 3 H). HRMS: m/z 677.8732 [C₃₇H₅₆F₃N₄O₄]⁺.

### According to the preparative method of the above examples, the following compounds 35 to 76 (corresponding to Example 35 to Example 76) were also prepared:

| Structure | Molecular formula and molecular weight in MS | Structure | Molecular formula and molecular weight in MS |
|---|---|---|---|
| 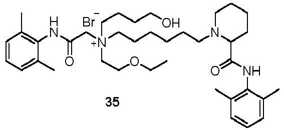 | HRMS: [C₃₈H₆₁N₄O₄]⁺ : 637.9292 | 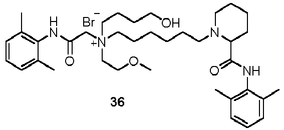 | HRMS: [C₃₇H₅₉N₄O₄]⁺ : 623.9021 |
| 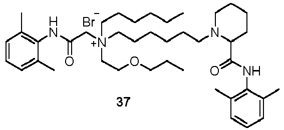 | HRMS: [C₄₁H₆₇N₄O₃]⁺ : 664.0117 | 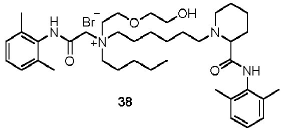 | HRMS: [C₃₉H₆₃N₄O₄]⁺ : 651.9566 |
| 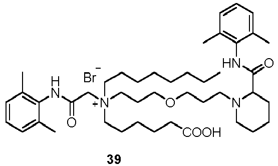 | HRMS: [C₄₄H₇₁N₄O₅]⁺ : 736.0748 | 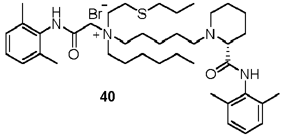 | HRMS: [C₄₀H₆₅N₄O₂]⁺ : 666.0441 |
| 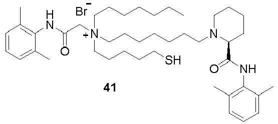 | HRMS: [C₄₃H₇₁N₄O₂S]⁺ : 708.1269 | 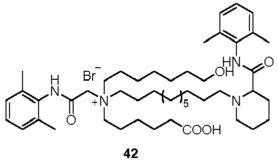 | HRMS: [C₄₈H₇₉N₄O₅]⁺ : 792.0740 |
| 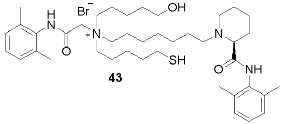 | HRMS: [C₄₁H₆₇N₄O₃S]⁺ : 696.0718 | 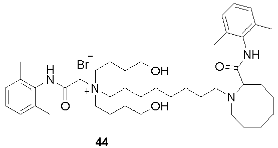 | HRMS: [C₄₂H₆₉N₄O₄]⁺ : 694.0377 |
| 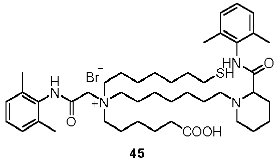 | HRMS: [C₄₄H₇₁N₄O₄S]⁺ : 752.1359 | 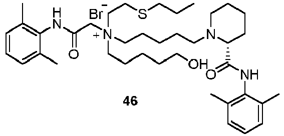 | HRMS: [C₃₉H₆₃N₄O₃S]⁺ : 668.0171 |
| 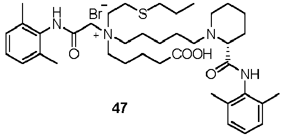 | HRMS: [C₃₉H₆₁N₄O₄S]⁺ : 681.4411 | 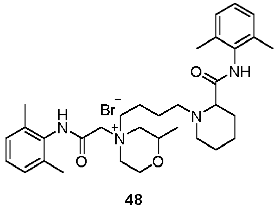 | HRMS: [C₃₃H₄₉N₄O₃]⁺ : 549.3795 |
| 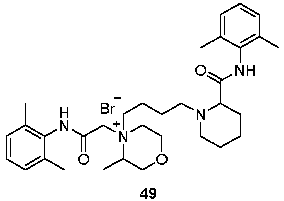 | HRMS: [C₃₃H₄₉N₄O₃]⁺ : 549.3798 | 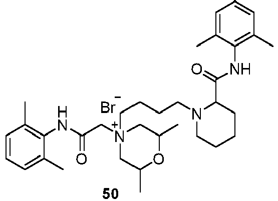 | HRMS: [C₃₄H₅₁N₄O₃]⁺ : 563.3959 |
| 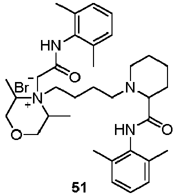 | HRMS: [C₃₄H₅₁N₄O₃]⁺ : 563.3952 | 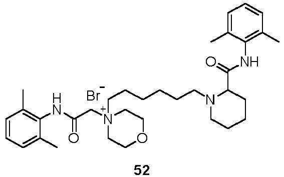 | HRMS: [C₃₄H₅₁N₄O₃]⁺ : 563.3953 |
| 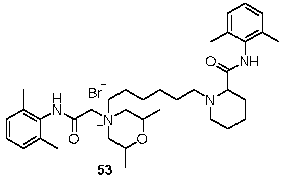 | HRMS: [C₄₁H₆₇N₄O₃]⁺ : 663.5203 | 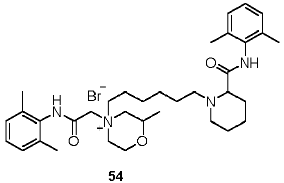 | HRMS: [C₃₅H₅₃N₄O₃]⁺ : 577.4116 |
| 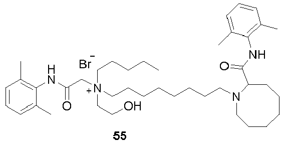 | HRMS: [C₃₆H₅₅N₄O₃]⁺ : 591.4263 | 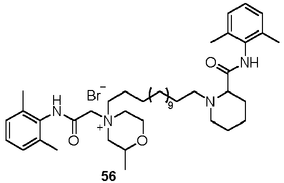 | HRMS: [C₄₃H₆₉N₄O₃]⁺ : 689.4112 |
| 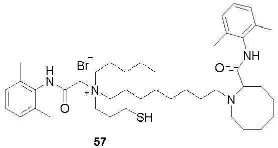 | HRMS: [C₄₂H₆₉N₄O₂S]⁺ : 693.5132 | 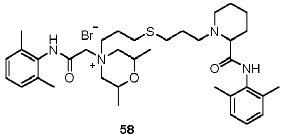 | HRMS: [C₃₆H₅₅N₄O₃S]⁺ : 623.3985 |
| 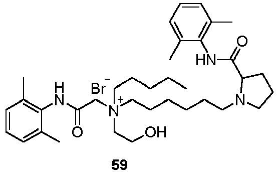 | HRMS: [C₃₆H₅₇N₄O₃]⁺ : 593.4428 | 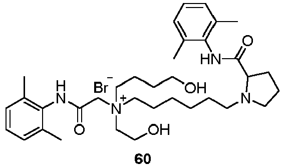 | HRMS: [C₃₅H₅₅N₄O₄]⁺ : 595.4214 |
| 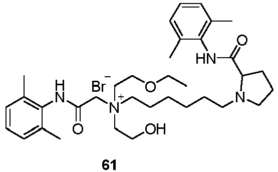 | HRMS: [C₃₅H₅₅N₄O₄]⁺ : 595.4215 | 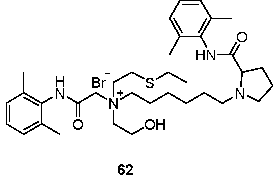 | HRMS: [C₃₅H₅₅N₄O₃S]⁺ : 611.3988 |
| 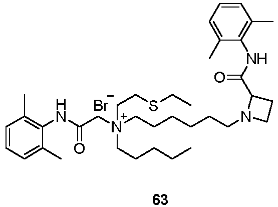 | HRMS: [C₃₇H₅₉N₄O₂S]⁺ : 623.4359 | 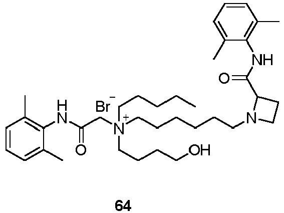 | HRMS: [C₃₇H₅₉N₄O₃]⁺ : 607.4587 |
| 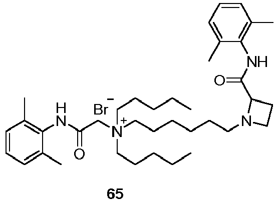 | HRMS: [C₃₈H₆₁N₄O₂]⁺ : 605.4783 | 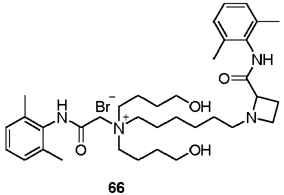 | HRMS: [C₃₆H₅₇N₄O₄]⁺ : 609.4377 |
| 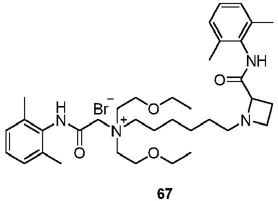 | HRMS: [C₃₆H₅₇N₄O₄]⁺ : 609.4379 | 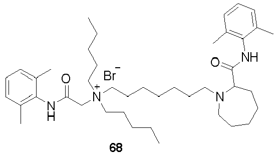 | HRMS: [C₄₂H₆₉N₄O₂]⁺ : 661.5411 |
| 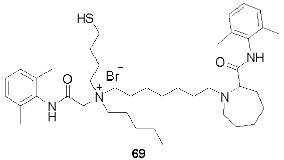 | HRMS: [C₄₁H₆₇N₄O₂S]⁺ : 679.4972 | 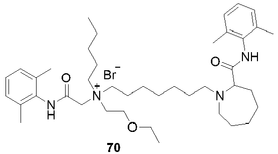 | HRMS: [C₄₁H₆₇N₄O₃]⁺ : 663.5213 |
| 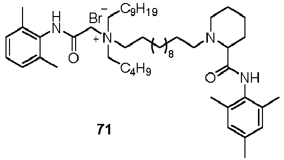 | HRMS: [C₄₅H₇₅N₄O₄]⁺ : 735.9292 | 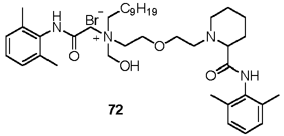 | HRMS: [C₃₉H₆₃N₄O₄]⁺ : 651.4840 |
| 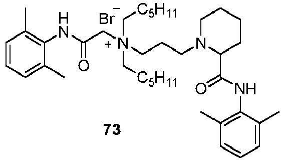 | HRMS: [C₃₉H₆₃N₄O₂]⁺ : 619.4942 | 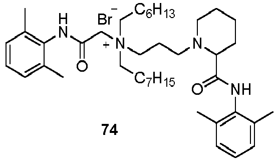 | HRMS: [C₄₂H₆₉N₄O₂]⁺ : 661.5418 |
| 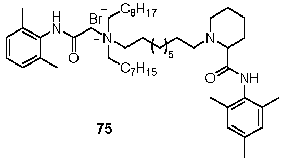 | HRMS: [C₅₁H₆₉N₄O₄]⁺ : 693.9292 | 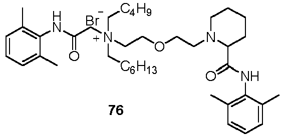 | HRMS: [C₄₀H₆₅N₄O₄₃]⁺ : 649.5055 |

In the following, the beneficial effects of the compounds of the present invention are illustrated by experimental examples.

### Experimental example 1. Study on the local anesthetic effect of the compounds of the present invention

Selected compounds **1-76** prepared in Examples, lidocaine (the positive control group), and levobupivacaine (the positive control group) were respectively assigned SD rats weighing 250-300 g (half male and half female), and the rats were fully adapted to the experimental environment, 8 rats for each group.

Dosage: for lidocaine group, lidocaine was formulated with distilled water into a solution at the concentration of 2% (74 mmol/L); for levobupivacaine group, levobupivacaine was formulated with distilled water into a solution at the concentration of 0.75% (22 mmol/L); the compound of the present invention was formulated with distilled water into a solution at the concentration of 20 mmol/mL.

The injection volume for each rat was 0.5 mL, which was guided by a nerve locator and injected near the rat's sciatic nerve. Using von Frey stimulator, the rats were stimulated the soles of the feet in the injected side, to observe the effect of local anesthesia. Meanwhile, Postural Extensor Thrust (PET) was used to evaluate the motor function of the rat: the rat was lifted vertically and the hindlimb in the injected side pedaled on the platform of the electronic balance. At this time, the muscle strength of the rat's hindlimb was displayed by the value on the balance caused by pedal. When the limb was completely paralyzed, the reading was the limb's own weight, about 20 g. If the measured value was more than half of the difference between the baseline and the limb weight, the motor function was regarded as recovery, and if the value was less than or equal to this difference, the motor function was regarded as loss.

**Table 1. Local anesthetic effect of the compound according to the present invention**

| **Test drug** | **Local anesthesia onset time** | **Duration of sensory block** | **Duration of motor block** |
|---|---|---|---|
| Example 1 product | 3 min | 32 h | 21 h |
| Example 2 product | 4 min | 44 h | 33 h |
| Example 3 product | 4 min | 35 h | 24 h |
| Example 4 product | 7 min | 23 h | 13 h |
| Example 5 product | 4 min | 26 h | 15 h |
| Example 6 product | 1 min | 34 h | 29 h |
| Example 7 product | 5 min | 26 h | 16 h |
| Example 8 product | 4 min | 43 h | 18 h |
| Example 9 product | 4 min | 34 h | 15 h |
| Example 10 product | 3 min | 25 h | 19 h |
| Example 11 product | 5 min | 34 h | 16 h |
| Example 12 product | 5 min | 42 h | 11 h |
| Example 13 product | 4 min | 55 h | 14 h |
| Example 14 product | 5 min | 41 h | 13 h |
| Example 15 product | 5 min | 44 h | 16 h |
| Example 16 product | 5 min | 60 h | 17 h |
| Example 17 product | 5 min | 37 h | 18 h |
| Example 18 product | 2 min | 38 h | 16 h |
| Example 19 product | 5 min | 39 h | 10 h |
| Example 20 product | 5 min | 54 h | 14 h |
| Example 21 product | 5 min | 47 h | 13 h |
| Example 22 product | 3 min | 47 h | 15 h |
| Example 23 product | 5 min | 54 h | 14 h |
| Example 24 product | 4 min | 54 h | 11 h |
| Example 25 product | 5 min | 44 h | 12 h |
| Example 26 product | 5 min | 45 h | 16 h |
| Example 27 product | 3 min | 37 h | 15 h |
| Example 28 product | 2 min | 39 h | 14 h |
| Example 29 product | 5 min | 50 h | 15 h |
| Example 31 product | 5 min | 45 h | 19 h |
| Example 32 product | 2 min | 46 h | 17 h |
| Example 33 product | 5 min | 49 h | 16 h |
| Example 34 product | 5 min | 45 h | 9 h |
| Example 35 product | 2 min | 54 h | 13 h |
| Example 36 product | 5 min | 52 h | 21 h |
| Example 37 product | 1 min | 45 h | 17 h |
| Example 38 product | 1 min | 43 h | 19 h |
| Example 39 product | 3 min | 61 h | 19 h |
| Example 40 product | 2 min | 53 h | 13 h |
| Example 41 product | 2 min | 46 h | 11 h |
| Example 42 product | 5 min | 56 h | 11 h |
| Example 43 product | 5 min | 59 h | 12 h |
| Example 44 product | 5 min | 69 h | 16 h |
| Example 45 product | 3 min | 58 h | 20 h |
| Example 46 product | 5 min | 49 h | 14 h |
| Example 47 product | 3 min | 65 h | 17 h |
| Example 48 product | 5 min | 57 h | 15 h |
| Example 49 product | 4 min | 45 h | 20 h |
| Example 50 product | 5 min | 56 h | 13 h |
| Example 51 product | 5 min | 44 h | 19 h |
| Example 52 product | 2 min | 56 h | 15 h |
| Example 53 product | 5 min | 59 h | 16 h |
| Example 54 product | 5 min | 66 h | 15 h |
| Example 55 product | 1 min | 55 h | 16 h |
| Example 56 product | 1 min | 54 h | 18 h |
| Example 57 product | 5 min | 69 h | 13 h |
| Example 58 product | 3 min | 57 h | 16 h |
| Example 59 product | 1 min | 54 h | 15 h |
| Example 60 product | 1 min | 54 h | 20 h |
| Example 61 product | 3 min | 6 1h | 11 h |
| Example 62 product | 5 min | 51 h | 21 h |
| Example 63 product | 3 min | 64 h | 14 h |
| Example 64 product | 3 min | 67 h | 13 h |
| Example 65 product | 5 min | 59 h | 19 h |
| Example 66 product | 1 min | 60 h | 10 h |
| Example 67 product | 1 min | 67 h | 14 h |
| Example 68 product | 1 min | 79 h | 21 h |
| Example 69 product | 1 min | 62h | 10 h |
| Example 70 product | 2 min | 53 h | 17 h |
| Example 71 product | 1 min | 60 h | 19 h |
| Example 72 product | 1 min | 65 h | 10 h |
| Example 73 product | 3 min | 51 h | 20 h |
| Example 74 product | 1 min | 72 h | 17 h |
| Example 75 product | 1 min | 61 h | 14 h |
| Example 76 product | 3 min | 59 h | 14 h |
| 0.75% levobupivacaine hydrochloride | 1 min | 2.5 h | 2.5 h |
| 2% lidocaine hydrochloride | 1 min | 1 h | 1 h |

Experimental results showed that the compounds of the present invention could produce local anesthesia lasting more than 24 h, and the blocking time of the sensory nerve was significantly longer than that of the motor nerve, and the difference time is greater than or equal to 5 h, wherein most of the compounds have a time difference of greater than or equal to 10 h.

### Experimental example 2. Local anesthetic effect of the compound according to the present invention by subcutaneous infiltration

After the back of SD rat weighing 250 to 300 g (half female and half male) was shaved and disinfected, a circle with a diameter of about 1.5 cm was drawn on the side of the exposed back, and the circle is divided into 6 equal parts. 0.5 mL solution containing a drug was subcutaneously injected into the skin of the center: using saline as the solvent, 0.75% bupivacaine hydrochloride (22 mmol/L), 2% lidocaine hydrochloride (74 mmol/L), the concentration of compounds **1-76** according to the present invention being 6 mmol/L, 10 rats for each group. Among the Von Frey fiber yarns, the one with a strength of 100 g was bound to the needle for local skin stimulation. One minute After the drug was injected, the above method was used to stimulate in 6 divisions. If no back skin contraction behavior was observed in the same aliquot after three consecutive stimulations, the drug was considered to have positive effect. If back skin contraction was observed, the local anesthetic effect was considered as loss. If four or more areas in 6 aliquots showed positive local anesthesia, the local anesthesia of the drug was considered as effective, while if less than 4 areas in 6 aliquots showed positive, the local anesthesia was considered as failure. Each compound was tested with 10 rats.

**Table 2. Local anesthetic effect of the compound according the present invention by subcutaneous infiltration.**

| **Test drug** | **Local anesthesia onset time (median)** | **Duration of local anesthesia (median)** |
|---|---|---|
| Example 1 product | 1 min | 25 h |
| Example 2 product | 2 min | 44 h |
| Example 3 product | 1 min | 38 h |
| Example 4 product | 1 min | 35 h |
| Example 5 product | 1 min | 56 h |
| Example 6 product | 3 min | 43 h |
| Example 7 product | 1 min | 56 h |
| Example 8 product | 1 min | 43 h |
| Example 9 product | 2 min | 38 h |
| Example 10 product | 1 min | 56 h |
| Example 11 product | 2 min | 39 h |
| Example 12 product | 3 min | 66 h |
| Example 13 product | 4 min | 53 h |
| Example 14 product | 2 min | 75 h |
| Example 15 product | 4 min | 73 h |
| Example 16 product | 2 min | 54 h |
| Example 17 product | 2 min | 56 h |
| Example 18 product | 1 min | 49 h |
| Example 19 product | 1 min | 62 h |
| Example 20 product | 3 min | 47 h |
| Example 21 product | 1 min | 64 h |
| Example 22 product | 2 min | 63 h |
| Example 23 product | 2 min | 83 h |
| Example 24 product | 2 min | 89 h |
| Example 25 product | 3 min | 82 h |
| Example 26 product | 3 min | 92 h |
| Example 27 product | 3 min | 71 h |
| Example 28 product | 3 min | 83 h |
| Example 29 product | 2 min | 73 h |
| Example 31 product | 1 min | 62 h |
| Example 32 product | 2 min | 56 h |
| Example 33 product | 1 min | 89 h |
| Example 34 product | 1 min | 76 h |
| Example 35 product | 2 min | 92 h |
| Example 36 product | 1 min | 81 h |
| Example 37 product | 1 min | 64 h |
| Example 38 product | 3 min | 66 h |
| Example 39 product | 2 min | 89 h |
| Example 40 product | 3 min | 76 h |
| Example 41 product | 3 min | 85 h |
| Example 42 product | 2 min | 73 h |
| Example 43 product | 2 min | 93 h |
| Example 44 product | 2 min | 51 h |
| Example 45 product | 1 min | 89 h |
| Example 46 product | 1 min | 76 h |
| Example 47 product | 1 min | 92 h |
| Example 48 product | 2 min | 73 h |
| Example 49 product | 1 min | 62 h |
| Example 50 product | 2 min | 56 h |
| Example 51 product | 1 min | 89 h |
| Example 52 product | 3 min | 85 h |
| Example 53 product | 1 min | 80 h |
| Example 54 product | 2 min | 69 h |
| Example 55 product | 3 min | 74 h |
| Example 56 product | 1 min | 101 h |
| Example 57 product | 1 min | 85 h |
| Example 58 product | 2 min | 71 h |
| Example 59 product | 1 min | 72 h |
| Example 60 product | 2 min | 66 h |
| Example 61 product | 1 min | 86 h |
| Example 62 product | 1 min | 88 h |
| Example 63 product | 1 min | 79 h |
| Example 64 product | 1 min | 69 h |
| Example 65 product | 1 min | 68 h |
| Example 66 product | 1 min | 87 h |
| Example 67 product | 1 min | 58 h |
| Example 68 product | 3 min | 73 h |
| Example 69 product | 1 min | 80 h |
| Example 70 product | 4 min | 75 h |
| Example 71 product | 1 min | 808 h |
| Example 72 product | 1 min | 72 h |
| Example 73 product | 2 min | 59 h |
| Example 74 product | 1 min | 56 h |
| Example 75 product | 1 min | 58 h |
| Example 76 product | 2 min | 64 h |
| 0.75% levobupivacaine Hydrochloride | 1 min | 7 h |
| 2% lidocaine hydrochloride | 1 min | 4 h |

Experimental results showed that this class of drugs could produce local anesthesia lasting more than 24 hours in the subcutaneous infiltration model of rat, wherein most compounds could produce local anesthesia for more than 40 hours.

### Experimental example 3. Evaluation of neuropathological damage of the compound according to the present invention

Selected compounds **1-76** prepared in Examples, lidocaine (the positive control group), and levobupivacaine (the positive control group) were respectively assigned SD rats weighing 250-300 g (half male and half female), and the rats were fully adapted to the experimental environment, 8 rats for each group.

Dosage: for lidocaine group, lidocaine was formulated with distilled water into a solution at the concentration of 2% (74 mmol/L); for levobupivacaine group, levobupivacaine was formulated with distilled water into a solution at the concentration of 0.75% (22 mmol/L); the compound of the present invention was formulated with distilled water into a solution at the concentration of 20 mmol/mL.

The injection volume for each rat was 0.5 mL, which was injected near the rat's sciatic nerve. On day 7 and day 14 after injection near the sciatic nerve, the experimental rats were euthanized by injecting bupivacaine into the heart under isoflurane anesthesia. About 1.5 cm sciatic nerve was collected at the injection site, stored in 10% formaldehyde solution for 48 hours, stained with HE, and cut into slices with 5 µm thickness.

Dosage: for lidocaine group, lidocaine was formulated with distilled water into a solution at the concentration of 2% (74 mmol/L); for levobupivacaine group, levobupivacaine was formulated with distilled water into a solution at the concentration of 0.75% (22 mmol/L); the compound of the present invention was formulated with distilled water into a solution at the concentration of 6 mmol/mL.

The injection volume for each rat was 0.5 mL, which was injected under the skin of the back of the rat. On day 7 and day 14 after subcutaneous injection, the experimental rats were euthanized by injecting bupivacaine into the heart under isoflurane anesthesia. The skin tissue at the injection site was collected, stored in 10% formaldehyde solution for 48 hours, stained with HE, and cut into slices with 5 µm thickness.

The evaluation of neuropathological damage showed that compared with the lidocaine positive control group and the levobupivacaine positive control group, the compounds of the Examples did not show significant differences in the aspects of nerve injury, vascular proliferation, demyelination, muscle inflammation, and connective tissue inflammation, and thus had good safety.

In summary, the present invention provides a new class of quaternary ammonium compounds with novel structures, as well as the preparation method and the use thereof. The compound had a fast onset of action and a long-time local anesthetic effect (more than 24 hours, and the local anesthesia time of most compounds exceeding 40 hours) after a single administration. The compound was selective for nerve block (the blocking time of sensory nerve was longer than that of motor nerve, and the difference time was greater than or equal to 5 hours, moreover, the difference time of most compounds was more than 10 hours), and had both long-acting and selective local anesthetic effect, that significantly reduced the side effects of QX314, QX314 compositions, and the quaternary ammonium compound with surfactant structure characteristics, with better safety. The compound of the present invention and its pharmaceutically acceptable salts could be used to prepare safe drugs with long-acting and selective local anesthesia, which had the advantages of long-time local anesthetic action, good nerve-selectivity for local anesthesia, less nerve damage, and high safety.

## Claims

1. A compound of formula I, or a pharmaceutically acceptable salt thereof, or a stereoisomer thereof, or a solvate thereof: 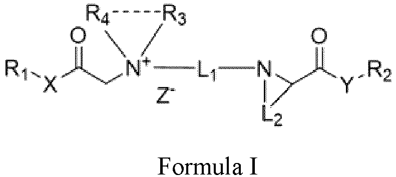 wherein,
each of X and Y is independently selected from the group consisting of O and NR₁₀, wherein R₁₀ is selected from the group consisting of H, deuterium, and C₁-C₄ alkyl;
Z⁻ is a pharmaceutically acceptable anion;
R₁ is n₁ R₁₁-substituted aryls;
R₂ is n₁' R₁₁'-substituted aryls;
wherein, n₁ and n₁' are each independently an integer of from 0 to 5, and R₁₁ and R₁₁' are each independently selected from the group consisting of deuterium, C₁-C₄ alkyl, C₁-C₄ alkoxy, halogen, nitro, cyano, hydroxyl, carboxyl, and amino;
when the dotted line between R₃ and R₄ in Formula I is none, R₃ is a substituted C₁-C₁₀ alkyl or unsubstituted C₅-C₁₀ alkyl, and R₄ is independently a substituted or unsubstituted C₁-C₁₀ alkyl, wherein said substituent is selected from the group consisting of deuterium, substituted or unsubstituted C₁-C₄ alkoxy, halogen, nitro, cyano, hydroxyl, carboxy, amino, ester, C₁-C₆ alkylthio, and mercapto; the substituent of said alkoxy is an hydroxyl;
when the dotted line between R₃ and R₄ in formula I is a bond, R₃ and R₄ are independently substituted or unsubstituted C₁-C₄ alkylenes, and the substituent is C₁-C₃ alkyl; wherein, the main chain of the alkylene contains 0 to 4 heteroatoms, and the heteroatom is selected from the group consisting of O, S, and NR₁₂, wherein said R₁₂ is selected from the group consisting of hydrogen, deuterium, and C₁-C₄ alkyl;
L₁ is a substituted or unsubstituted C₁-C₁₄ alkylenyl; wherein the main chain of the alkylene contains 0 to 4 heteroatoms, and the heteroatom is selected from the group consisting of O, S, and NR₁₂, wherein said R₁₂ is selected from the group consisting of hydrogen, deuterium, C₁-C₄ alkyl, and C₁-C₄ alkoxy; the substituent is selected from the group consisting of deuterium, C₁-C₄ alkyl, C₁-C₄ alkoxy, and halogen;
and L₂ is a substituted or unsubstituted C₁-C₈ alkylenyl, and the substituent is selected from the group consisting of deuterium, C₁-C₄ alkyl, C₁-C₄ alkoxy, and halogen.

2. The compound according to claim 1, or a pharmaceutically acceptable salt thereof, or a stereoisomer thereof, or a solvate thereof, **characterized in that**:
each of X and Y is independently NH or NCH₃;
Z⁻ is Br⁻,Cl⁻or sulfonate;
R₁ is n₁ R₁₁-substituted aryls;
R₂ is n₁' R₁₁'-substituted aryls;
wherein, each of n₁ and n₁' is independently an integer of from 0 to 5, and each of R₁₁ and R₁₁' is independently selected from the group consisting of deuterium, C₁-C₄ alkyl, C₁-C₄ alkoxy, halogen, nitro, cyano, and hydroxyl;
when the dotted line between R₃ and R₄ in Formula I is none, R₃ is a substituted C₁-C₁₀ alkyl or unsubstituted C₅-C₁₀ alkyl, and R₄ is independently a substituted or unsubstituted C₁-C₁₀ alkyl, wherein said substituent is selected from the group consisting of deuterium, substituted or unsubstituted C₁-C₄ alkoxy, hydroxyl, carboxy, C₂-C₅ alkylthio, and mercapto; the substituent of said alkoxy is an hydroxyl;
when the dotted line between R₃ and R₄ in formula I is a bond, R₃ and R₄ are independently C₁-C₄ alkylenyls, wherein the main chain of the alkylene contains 0 to 2 heteroatoms, and the heteroatom is O;
L₁ is a C₃-C₁₄ alkylenyl; wherein the substituent is alkyl, and the main chain of the alkylene contains 0 to 2 heteroatoms, and the heteroatom is selected from the group consisting of O and S;
and L₂ is a C₁-C₆ alkylenyl.

3. The compound according to claim 1, or a pharmaceutically acceptable salt thereof, or a stereoisomer thereof, or a solvate thereof, **characterized in that**:
each of X and Y is independently NH or NCH₃;
Z⁻ is Br⁻, Cl⁻ or sulfonate;
R₁ is n₁ R₁₁-substituted aryls;
R₂ is n₁' R₁₁'-substituted aryls;
wherein, each of n₁ and n₁' is independently an integer of from 2 to 3, and R₁₁ and R₁₁' are independently selected from the group consisting of methyl, propyl, methoxy, hydroxy, nitro, cyano, and halogen;
when the dotted line between R₃ and R₄ in Formula I is none, R₃ is a substituted C₁-C₈ alkyl or unsubstituted C₅-C₁₀ alkyl, and R₄ is independently a substituted or unsubstituted C₁-C₁₀ alkyl, and said substituent is selected from the group consisting of deuterium, substituted or unsubstituted C₁-C₃ alkoxy, hydroxyl, carboxy, C₂-C₅ alkylthio, and mercapto; the substituent of said alkoxy is an hydroxyl;
when the dotted line between R₃ and R₄ in formula I is a bond, R₃ and R₄ are independently substituted or unsubstituted C₂-C₃ alkylenyls, wherein the substituent is C₁ alkyl, and the main chain of the alkylene contains 0 to 1 heteroatom, and the heteroatom is O;
L₁ is a C₃-C₁₄ alkylenyl; wherein the main chain of the alkylene contains 0 to 2 heteroatoms, and the heteroatom is selected from the group consisting of O and S;
and L₂ is an unsubstituted C₁-C₆ alkylenyl

4. The compound according to claim 1, or a pharmaceutically acceptable salt thereof, or a stereoisomer thereof, or a solvate thereof, **characterized in that**:
each of X and Y is NH;
Z⁻ is Br⁻;
R₁ is n₁ R₁₁-substituted aryls;
R₂ is n₁' R₁₁'-substituted aryls;
wherein each of n₁ and n₁' is independently an integer of from 2 to 3, and R₁₁ and R₁₁' are each a methyl group;
when the dotted line between R₃ and R₄ in Formula I is none, R₃ is a substituted C₁-C₈ alkyl or
unsubstituted C₅-C₁₀ alkyl; said substituent is selected from the group consisting of deuterium,
substituted or unsubstituted C₁-C₃ alkoxy, hydroxyl, carboxy, C₂-C₃ alkylthio, and mercapto;
the substituent of said alkoxy is an hydroxyl;
when the dotted line between R₃ and R₄ in formula I is a bond, R₃ and R₄ are independently an unsubstituted C₂-C₃ alkylenyl; wherein the main chain of the alkylene contains one heteroatom,
and the heteroatom is O;
L₁ is an unsubstituted C₃-C₁₄ alkylenyl; wherein the main chain of the alkylene contains one heteroatom, and the heteroatom is selected from the group consisting of O and S;
and L₂ is an unsubstituted C₂-C₆ alkylenyl

5. The compound according to claim 1, or a pharmaceutically acceptable salt thereof, or a stereoisomer thereof, or a solvate thereof, **characterized in that** said compound is as shown in formula II: 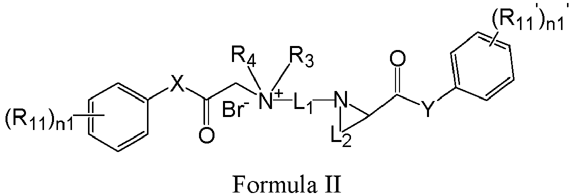 wherein
each of X and Y is independently NH or NCH₃;
each of n₁ and n₁' is independently an integer of from 2 to 3, and R₁₁ and R₁₁' are selected from the group consisting of methyl, propyl, methoxy, hydroxy, nitro, cyano, and halogen;
R₃ is a substituted C₁-C₈ alkyl or unsubstituted C₅-C₁₀ alkyl; R₄ is independently a substituted or unsubstituted C₁-C₁₀ alkyl; the substituent of said alkyl is selected from the group consisting of deuterium, substituted or unsubstituted C₁-C₃ alkoxy, hydroxyl, carboxy, C₂-C₅ alkylthio, and mercapto; the substituent of said alkoxy is an hydroxyl;
L₁ is an unsubstituted C₃-C₁₄ alkylenyl; wherein the main chain of the alkylene contains 0, 1 or 2 heteroatoms, and the heteroatom is selected from the group consisting of O and S;
L₂ is an unsubstituted C₂-C₆ alkylenyl.

6. The compound according to claim 5, or a pharmaceutically acceptable salt thereof, or a stereoisomer thereof, or a solvate thereof, **characterized in that** said compound is as shown in formula III: 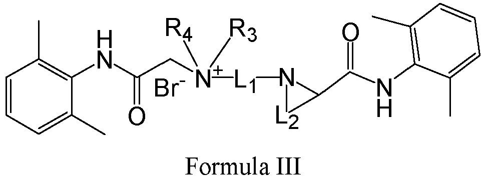 wherein
R₃ is a substituted C₁-C₅ alkyl; R₄ is independently a substituted or unsubstituted C₁-C₅ alkyl; the substituent of said alkyl is an hydroxyl;
L₁ is an unsubstituted C₃-C₆ alkylenyl;
L₂ is an unsubstituted C₄ alkylenyl.

7. The compound according to claim 1, or a pharmaceutically acceptable salt thereof, or a stereoisomer thereof, or a solvate thereof, **characterized in that** said compound is as shown in formula IV: 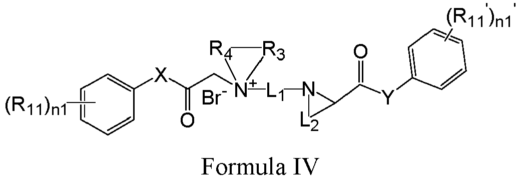 wherein
each of X and Y is NH;
each of n₁ and n₁' is independently an integer of from 2 to 3; Rn and R₁₁' are each a methyl group;
R₃ and R₄ are independently substituted or unsubstituted C₂-C₃ alkylenyls; wherein said substituent is C₁ alkyl, and the main chain of the alkylene contains one heteroatom, and the heteroatom is O;
L₁ is an unsubstituted C₃-C₁₄ alkylenyl; wherein the main chain of the alkylene contains 0 or 1 heteroatom, and the heteroatom is selected from the group consisting of O and S;
L₂ is an unsubstituted C₂-C₆ alkylenyl.

8. The compound according to any one of claims 1 to 7, or a pharmaceutically acceptable salt thereof, or a stereoisomer thereof, or a solvate thereof, **characterized in that** the structure of the compound is one of the following:

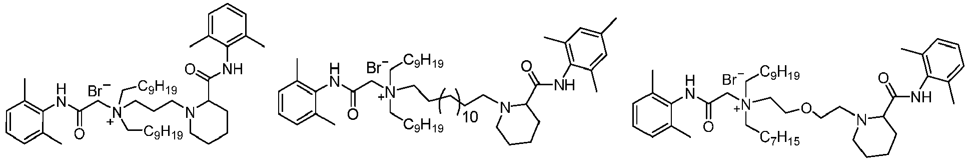

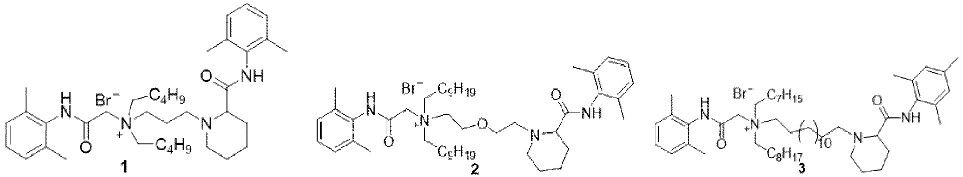

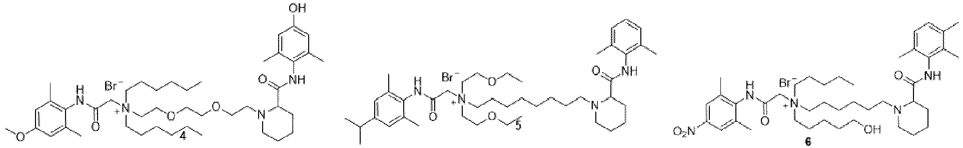

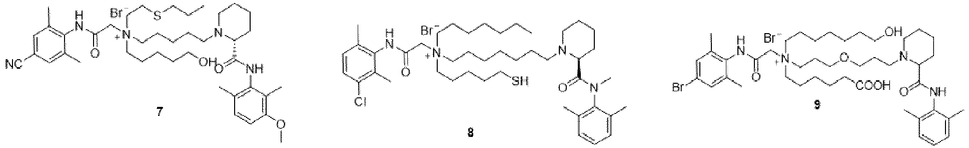

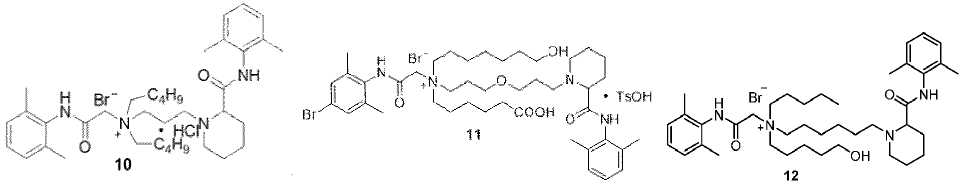

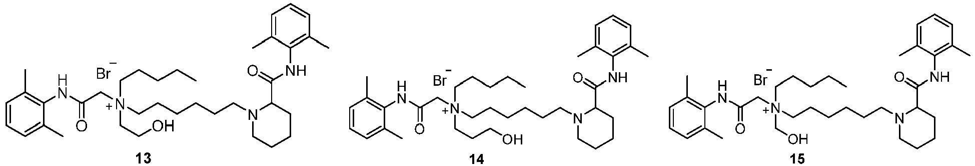

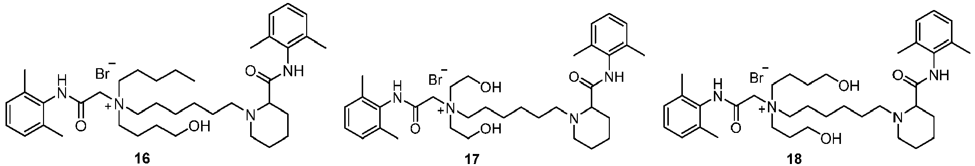

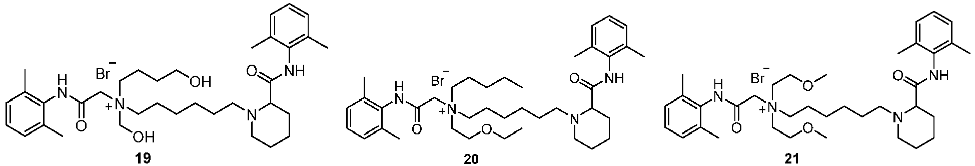

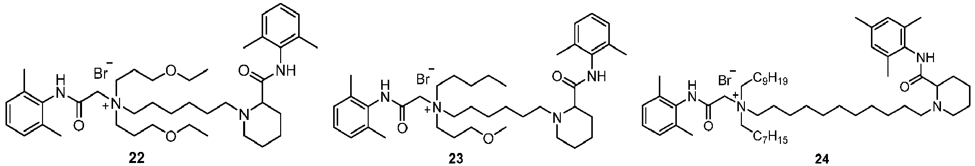

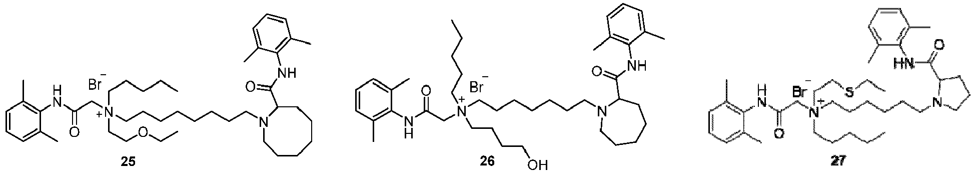

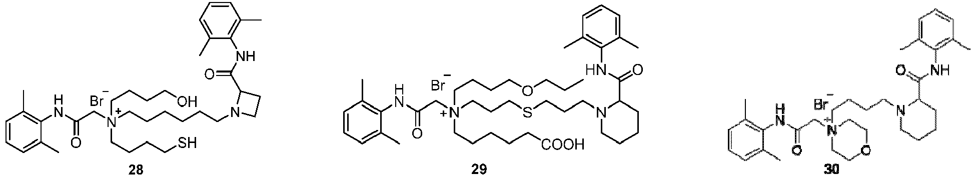

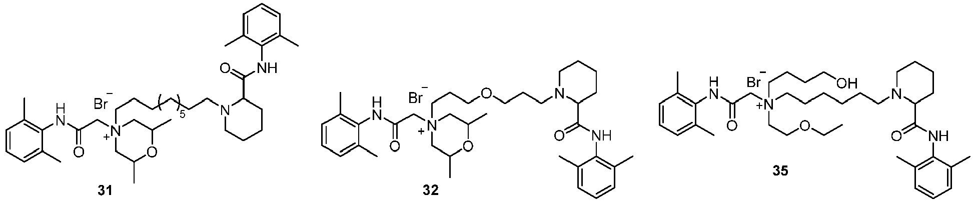

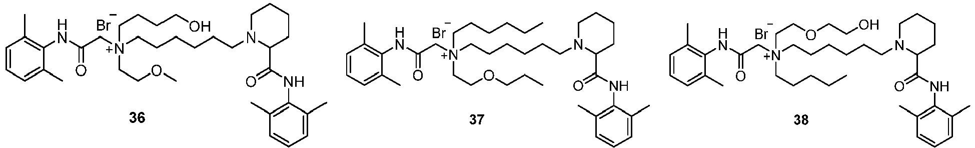

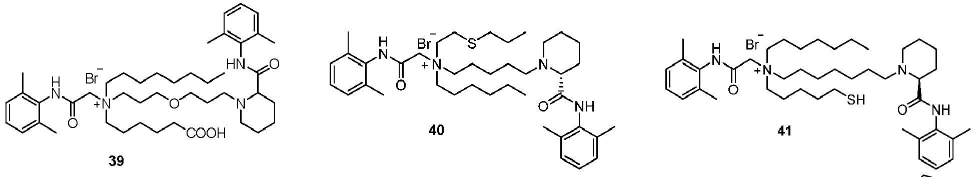

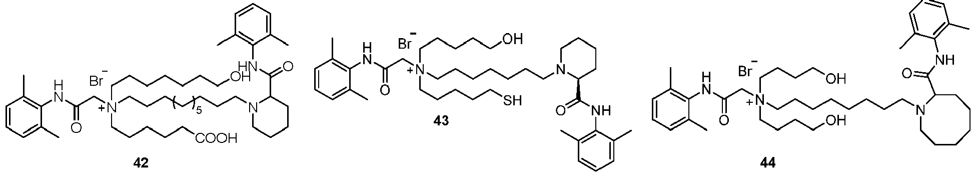

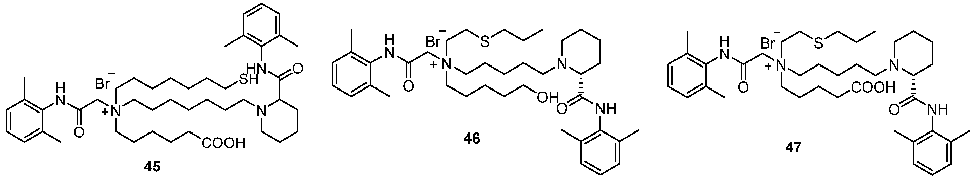

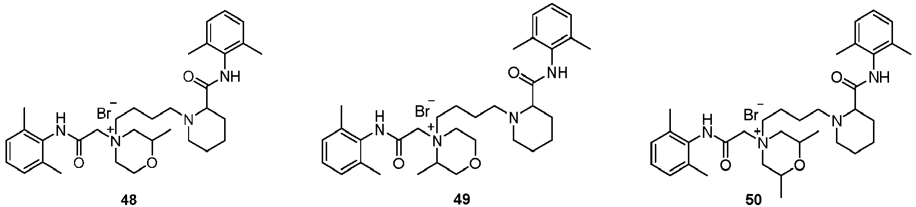

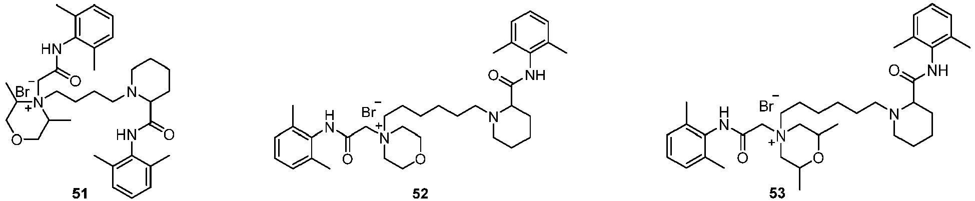

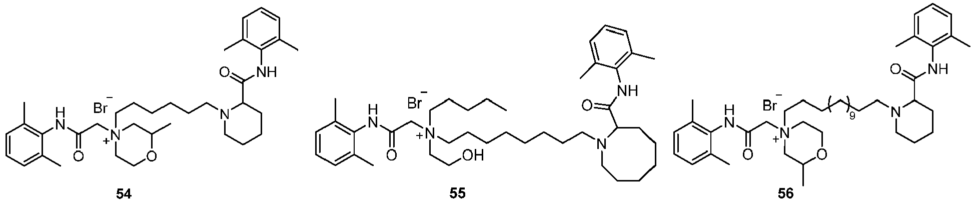

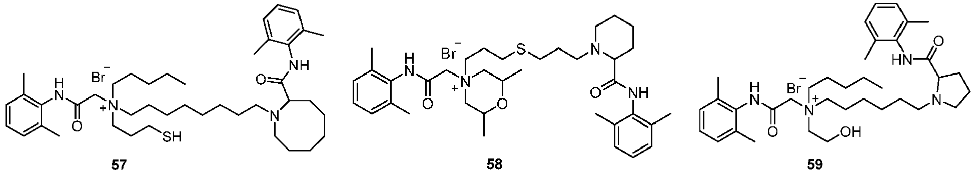

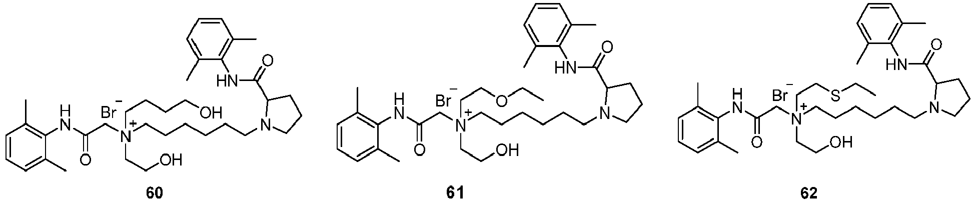

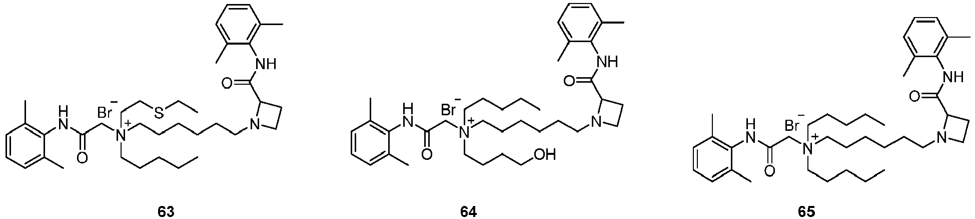

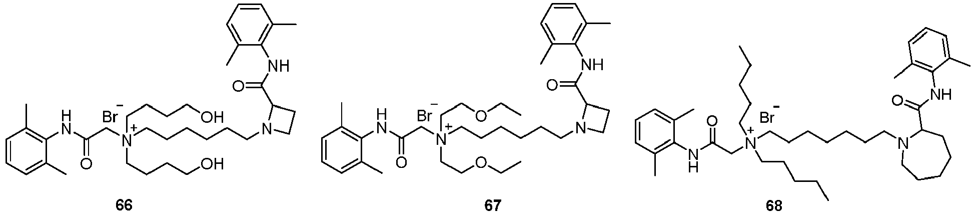

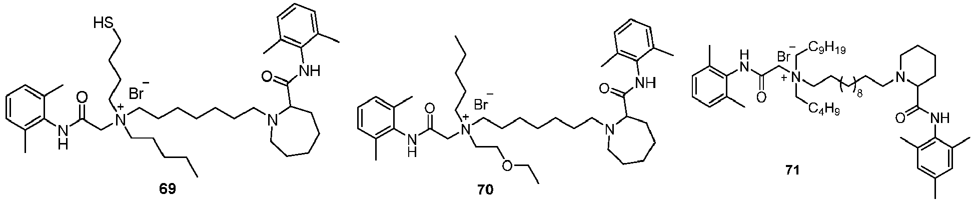

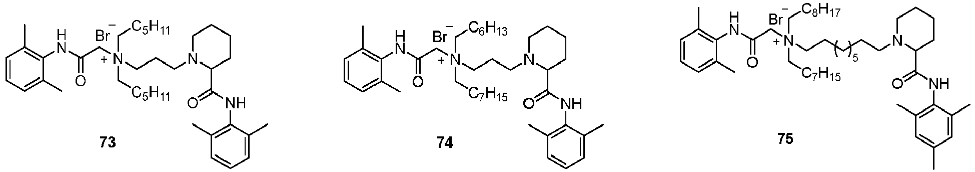

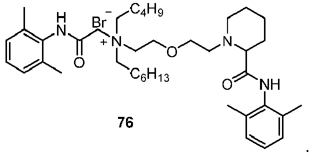

9. A compound according to any one of claims 1 to 8, or a pharmaceutically acceptable salt thereof, or a stereoisomer thereof, or a solvate threreof, for use as a local anesthetic medicine, wherein said compound is used together with a pharmaceutically acceptable carrier.

10. The compound for use according to claim 9, or a pharmaceutically acceptable salt thereof, or a stereoisomer thereof, or a solvate threreof, **characterized in that**
said local anesthetic medicine makes the blocking time of sensory nerve longer than that of motor nerve; and/or
said local anesthesia is long-acting local anesthesia;
preferably the anesthesia time of said local anesthesia exceeds 24 hours.

11. A drug, **characterized in that** it is a composition formed by a compound according to any one of claims 1 to 8, or a pharmaceutically acceptable salt thereof, or a stereoisomer thereof, or a solvate thereof, with the addition of pharmaceutically acceptable excipients.

## Patentansprüche

1. Verbindung der Formel I oder ein pharmazeutisch unbedenkliches Salz davon oder ein Stereoisomer davon oder ein Solvat davon: 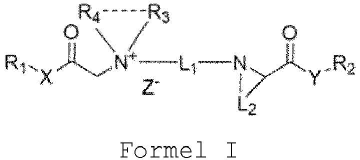 wobei
X und Y jeweils unabhängig aus der Gruppe bestehend aus O und NR₁₀ ausgewählt sind, wobei
R₁₀ aus der Gruppe bestehend aus H, Deuterium und C₁-C₄-Alkyl ausgewählt ist;
Z⁻ für ein pharmazeutisch unbedenkliches Anion steht;
R₁ für a n₁ R₁₁-substituierte Arylgruppen steht;
R₂ für n₁' R₁₁'-substituierte Arylgruppen steht;
wobei n₁ und n₁' jeweils unabhängig für eine ganze Zahl von 0 bis 5 stehen und R₁₁ und R₁₁' jeweils unabhängig aus der Gruppe bestehend aus Deuterium, C₁-C₄ -Alkyl, C₁-C₄-Alkoxy, Halogen, Nitro, Cyano, Hydroxyl, Carboxyl und Amino ausgewählt sind;
wenn die gestrichelte Linie zwischen R₃ und R₄ in Formel I für nichts steht, R₃ für ein substituiertes C₁-C₁₀-Alkyl oder unsubstituiertes C₅-C₁₀-Alkyl steht und R₄ unabhängig für ein substituiertes oder unsubstituiertes C₁-C₁₀-Alkyl steht, wobei der Substituent aus der Gruppe bestehend aus Deuterium, substituiertem oder unsubstituiertem C₁-C₄-Alkoxy, Halogen, Nitro, Cyano, Hydroxyl, Carboxy, Amino, Ester, C₁-C₆-Alkylthio und Mercapto ausgewählt ist; der Substituent des Alkoxys ein Hydroxyl ist;
wenn die gestrichelte Linie zwischen R₃ and R₄ in Formel I für eine Bindung steht, R₃ und R₄ unabhängig für substituierte oder unsubstituierte C₁-C₄-Alkylengruppen stehen und der Substituent C₁-C₃-Alkyl ist; wobei die Hauptkette des Alkylens 0 bis 4 Heteroatome enthält und das Heteroatom aus der Gruppe bestehend aus O, S und NR₁₂ ausgewählt ist, wobei das R₁₂ aus der Gruppe bestehend aus Wasserstoff, Deuterium und C₁-C₄-Alkyl ausgewählt ist;
L₁ für ein substituiertes oder unsubstituiertes C₁-C₁₄-Alkylenyl steht; wobei die Hauptkette des Alkylens 0 bis 4 Heteroatome enthält und das Heteroatom aus der Gruppe bestehend aus O, S und NR₁₂ ausgewählt ist, wobei das R₁₂ aus der Gruppe bestehend aus Wasserstoff, Deuterium, C₁-C₄-Alkyl und C₁-C₄-Alkoxy ausgewählt ist; der Substituent aus der Gruppe bestehend aus Deuterium, C₁-C₄-Alkyl, C₁-C₄-Alkoxy und Halogen ausgewählt ist;
und L₂ für ein substituiertes oder unsubstituiertes C₁-C₈-Alkylenyl steht und der Substituent aus der Gruppe bestehend aus Deuterium, C₁-C₄-Alkyl, C₁-C₄-Alkoxy und Halogen ausgewählt ist.

2. Verbindung nach Anspruch 1 oder ein pharmazeutisch unbedenkliches Salz davon oder ein Stereoisomer davon oder ein Solvat davon, **dadurch gekennzeichnet, dass**:
X und Y jeweils unabhängig für NH oder NCH₃ stehen;
Z⁻ für Br⁻, Cl⁻ oder Sulfonat steht;
R₂ für n₁ R₁₁-substituierte Arylgruppen steht;
R₂ für n₁' R₁₁'-substituierte Arylgruppen steht;
wobei n₁ und n₁' jeweils unabhängig für eine ganze Zahl von 0 bis 5 stehen und R₁₁ und R₁₁' jeweils unabhängig aus der Gruppe bestehend aus Deuterium, C₁-C₄-Alkyl, C₁-C₄-Alkoxy, Halogen, Nitro, Cyano und Hydroxyl ausgewählt sind;
wenn die gestrichelte Linie zwischen R₃ und R₄ in Formel I für nichts steht, R₃ für ein substituiertes C₁-C₁₀-Alkyl oder unsubstituiertes C₅-C₁₀-Alkyl steht und R₄ unabhängig für ein substituiertes oder unsubstituiertes C₁-C₁₀-Alkyl steht, wobei der Substituent aus der Gruppe bestehend aus Deuterium, substituiertem oder unsubstituiertem C₁-C₄-Alkoxy, Hydroxyl, Carboxy, C₂-C₅-Alkylthio und Mercapto ausgewählt ist; der Substituent des Alkoxys ein Hydroxyl ist;
wenn die gestrichelte Linie zwischen R₃ and R₄ in Formel I für eine Bindung steht, R₃ und R₄ unabhängig für C₁-C₄-Alkylengruppen stehen; wobei die Hauptkette des Alkylens 0 bis 2 Heteroatome enthält und das Heteroatom O ist;
L₁ für ein C₃-C₁₄-Alkylenyl steht; wobei die Hauptkette des Alkylens 0 bis 2 Heteroatome enthält und das Heteroatom aus der Gruppe bestehend aus O und S ausgewählt ist;
und L₂ für ein C₁-C₆-Alkylenyl steht.

3. Verbindung nach Anspruch 1 oder ein pharmazeutisch unbedenkliches Salz davon oder ein Stereoisomer davon oder ein Solvat davon, **dadurch gekennzeichnet, dass**:
X und Y jeweils unabhängig für NH oder NCH₃ stehen;
Z⁻ für Br⁻, Cl⁻ oder Sulfonat steht;
R₃ für n₁ R₁₁-substituierte Arylgruppen steht;
R₂ für n₁' R₁₁'-substituierte Arylgruppen steht;
wobei n₁ und n₁' jeweils unabhängig für eine ganze Zahl von 2 bis 3 stehen und R₁₁ und R₁₁' jeweils unabhängig aus der Gruppe bestehend Methyl, Propyl, Methoxy, Hydroxy, Nitro, Cyano und Halogen ausgewählt sind;
wenn die gestrichelte Linie zwischen R₃ und R₄ in Formel I für nichts steht, R₃ für ein substituiertes C₁-C₈-Alkyl oder unsubstituiertes C₅-C₁₀-Alkyl steht und R₄ unabhängig für ein substituiertes oder unsubstituiertes C₁-C₁₀-Alkyl steht und der Substituent aus der Gruppe bestehend aus Deuterium, substituiertem oder unsubstituiertem C₁-C₃-Alkoxy, Hydroxyl, Carboxy, C₂-C₅-Alkylthio und Mercapto ausgewählt ist; der Substituent des Alkoxys ein Hydroxyl ist;
wenn die gestrichelte Linie zwischen R₃ and R₄ in Formel I für eine Bindung steht, R₃ und R₄ unabhängig für substituierte oder unsubstituierte C₂-C₃-Alkylengruppen stehen; wobei der Substituent C₁-Alkyl ist und die Hauptkette des Alkylens 0 bis 1 Heteroatom enthält und das Heteroatom O ist;
L₁ für ein C₃-C₁₄-Alkylenyl steht; wobei die Hauptkette des Alkylens 0 bis 2 Heteroatome enthält und das Heteroatom aus der Gruppe bestehend aus O und S ausgewählt ist;
und L₂ für ein unsubstituiertes C₁-C₆-Alkylenyl steht.

4. Verbindung nach Anspruch 1 oder ein pharmazeutisch unbedenkliches Salz davon oder ein Stereoisomer davon oder ein Solvat davon, **dadurch gekennzeichnet, dass**:
X und Y jeweils unabhängig für NH stehen;
Z⁻ für Br⁻ steht;
R₁ für n₁ R₁₁-substituierte Arylgruppen steht;
R₂ für n₁' R₁₁'-substituierte Arylgruppen steht;
wobei n₁ und n₁' jeweils unabhängig für eine ganze Zahl von 2 bis 3 stehen und R₁₁ und R₁₁' jeweils unabhängig für eine Methylgruppe stehen;
wenn die gestrichelte Linie zwischen R₃ und R₄ in Formel I für nichts steht, R₃ für ein substituiertes C₁-C₈-Alkyl oder unsubstituiertes C₅-C₁₀-Alkyl steht; wobei der Substituent aus der Gruppe bestehend aus Deuterium, substituiertem oder unsubstituiertem C₁-C₃-Alkoxy, Hydroxyl, Carboxy, C₂-C₃-Alkylthio und Mercapto ausgewählt ist; der Substituent des Alkoxys ein Hydroxyl ist;
wenn die gestrichelte Linie zwischen R₃ and R₄ in Formel I für eine Bindung steht, R₃ und R₄ unabhängig für ein unsubstituiertes C₂-C₃-Alkylen stehen; wobei die Hauptkette des Alkylens ein Heteroatom enthält und das Heteroatom O ist;
L₁ für ein unsubstituiertes C₃-C₁₄-Alkylenyl steht; wobei die Hauptkette ein Heteroatom enthält und das Heteroatom aus der Gruppe bestehend aus O und S ausgewählt ist;
und L₂ für ein unsubstituiertes C₁-C₆-Alkylenyl steht.

5. Verbindung nach Anspruch 1 oder ein pharmazeutisch unbedenkliches Salz davon oder ein Stereoisomer davon oder ein Solvat davon, **dadurch gekennzeichnet, dass** die Verbindung wie in Formel 11 gezeigt ist: 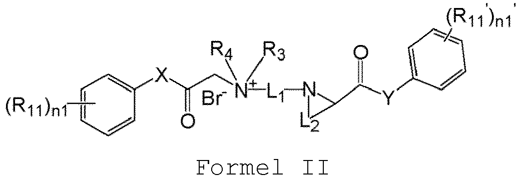 wobei
X und Y jeweils unabhängig für NH oder NCH₃ stehen;
n₁ und n₁' jeweils unabhängig für eine ganze Zahl von 2 bis 3 stehen und R₁₁ und R₁₁' jeweils unabhängig aus der Gruppe bestehend Methyl, Propyl, Methoxy, Hydroxy, Nitro, Cyano und Halogen ausgewählt sind;
R₃ für ein substituiertes C₁-C₈-Alkyl oder unsubstituiertes C₅-C₁₀-Alkyl steht; R₄ unabhängig für ein substituiertes oder unsubstituiertes C₁-C₁₀-Alkyl steht;
der Substituent des Alkyls aus der Gruppe bestehend aus Deuterium, substituiertem oder unsubstituiertem C₁-C₃-Alkoxy, Hydroxyl, Carboxy, C₂-C₅-Alkylthio und Mercapto ausgewählt ist; der Substituent des Alkoxys ein Hydroxyl ist;
L₁ für ein unsubstituiertes C₃-C₁₄-Alkylenyl steht; wobei die Hauptkette des Alkylens 0, 1 oder 2 Heteroatome enthält und das Heteroatom aus der Gruppe bestehend aus O und S ausgewählt ist;
L₂ für ein unsubstituiertes C₂-C₆-Alkylenyl steht.

6. Verbindung nach Anspruch 5 oder ein pharmazeutisch unbedenkliches Salz davon oder ein Stereoisomer davon oder ein Solvat davon, **dadurch gekennzeichnet, dass** die Verbindung wie in Formel III gezeigt ist: 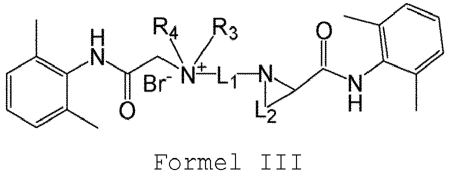 wobei
R₃ für ein substituiertes C₁-C₅-Alkyl steht; R₄ unabhängig für ein substituiertes oder unsubstituiertes C₁-C₅-Alkyl steht; der Substituent des Alkyls ein Hydroxyl ist;
L₁ für ein unsubstituiertes C₃-C₆-Alkylenyl steht;
L₂ für ein unsubstituiertes C₄-Alkylenyl steht.

7. Verbindung nach Anspruch 1 oder ein pharmazeutisch unbedenkliches Salz davon oder ein Stereoisomer davon oder ein Solvat davon, **dadurch gekennzeichnet, dass** die Verbindung wie in Formel IV gezeigt ist: 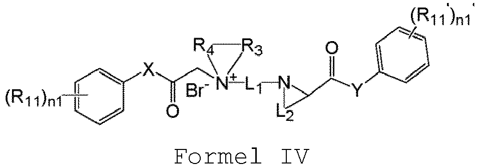 wobei
X und Y jeweils unabhängig für NH stehen;
n₁ und n₁' jeweils unabhängig für eine ganze Zahl von 2 bis 3 stehen und R₁₁ und R₁₁' jeweils unabhängig für eine Methylgruppe stehen;
R₃ und R₄ unabhängig für substituierte oder unsubstituierte C₂-C₃-Alkylenylgruppen stehen; wobei der Substituent C₁-Alkyl ist und die Hauptkette des Alkylens ein Heteroatom enthält und das Heteroatom O ist;
L₁ für ein unsubstituiertes C₃-C₁₄-Alkylenyl steht; wobei die Hauptkette 0 oder 1 Heteroatom enthält und das Heteroatom aus der Gruppe bestehend aus O und S ausgewählt ist;
L₂ für ein unsubstituiertes C₂-C₆-Alkylenyl steht.

8. Verbindung nach einem der Ansprüche 1 bis 7 oder ein pharmazeutisch unbedenkliches Salz davon oder ein Stereoisomer davon oder ein Solvat davon, **dadurch gekennzeichnet, dass** die Verbindung eine der folgenden Strukturen aufweist:

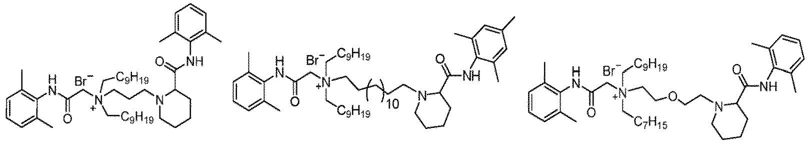

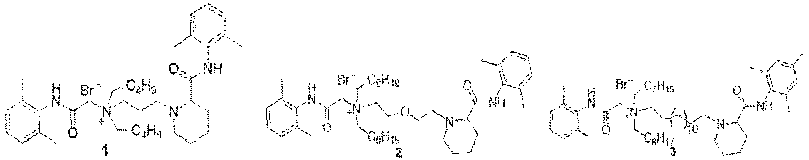

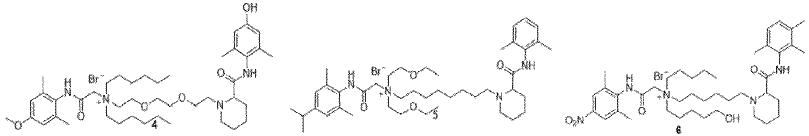

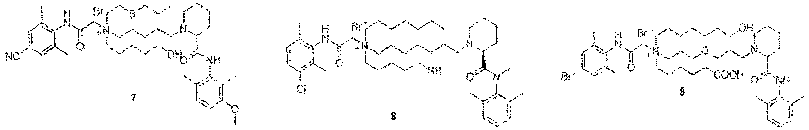

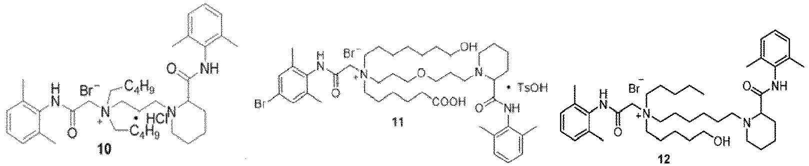

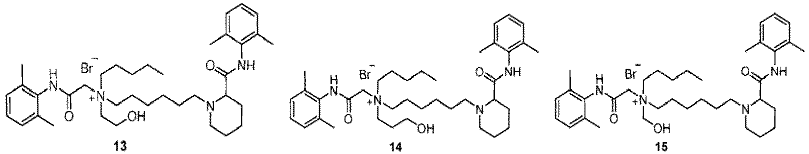

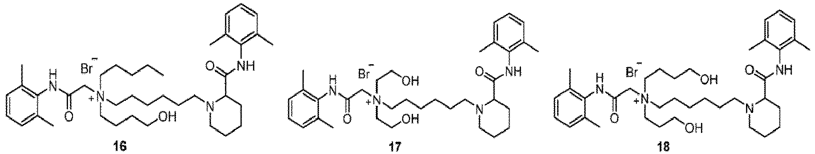

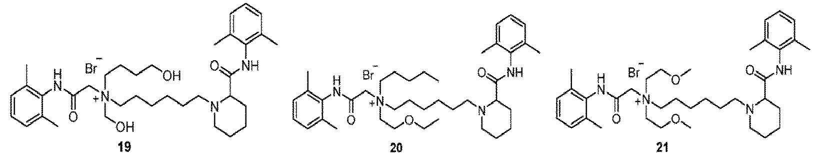

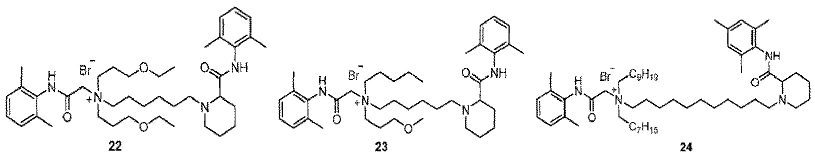

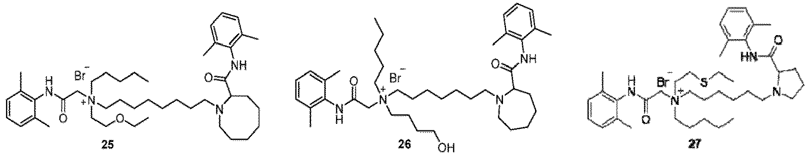

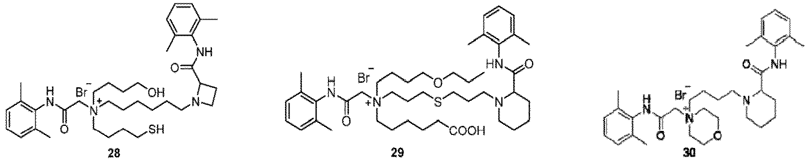

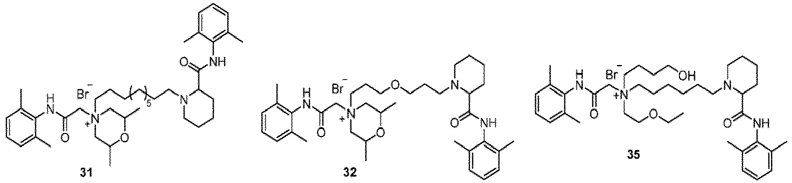

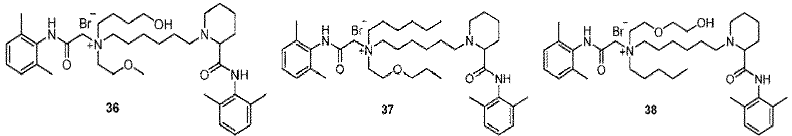

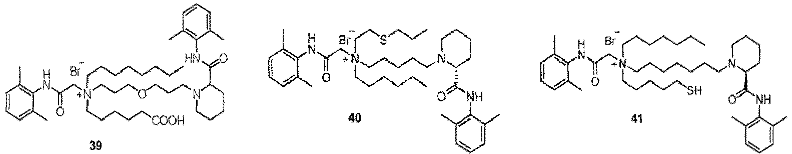

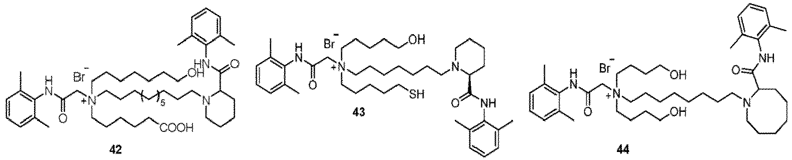

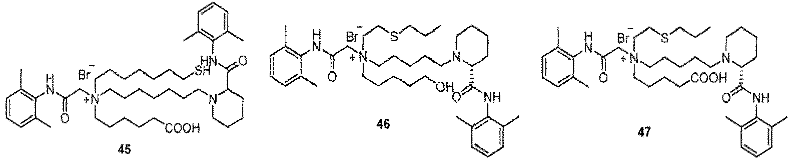

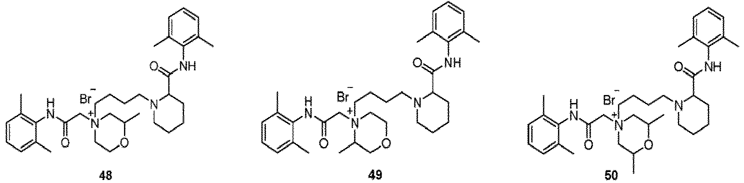

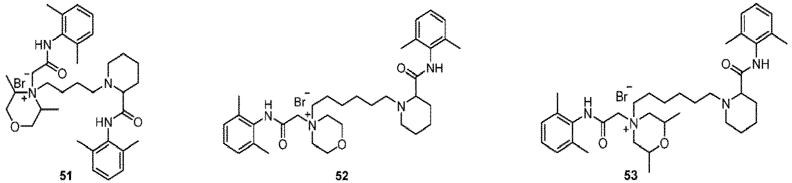

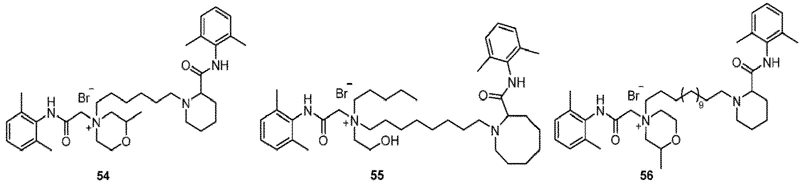

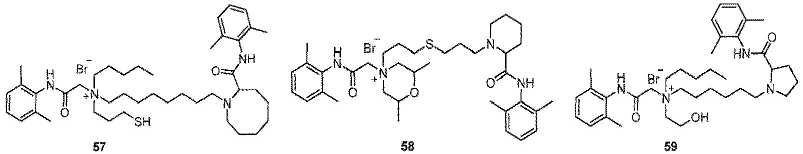

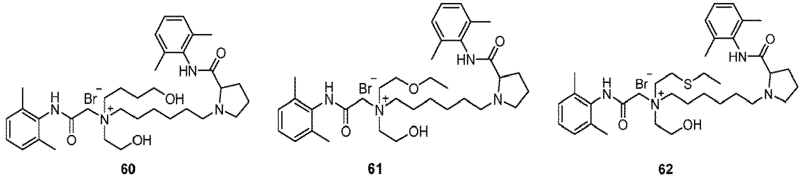

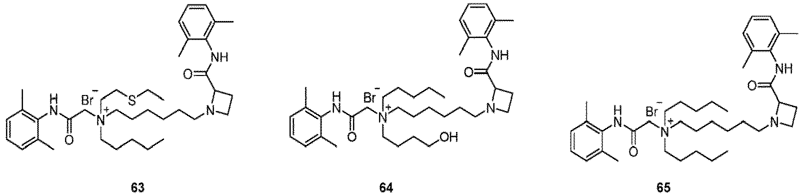

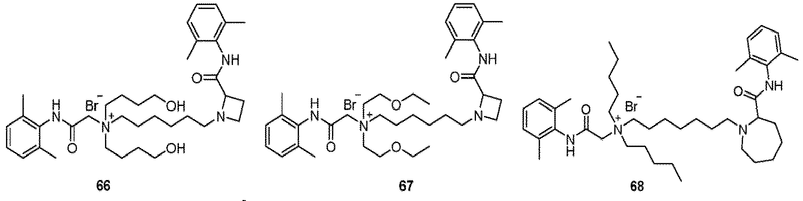

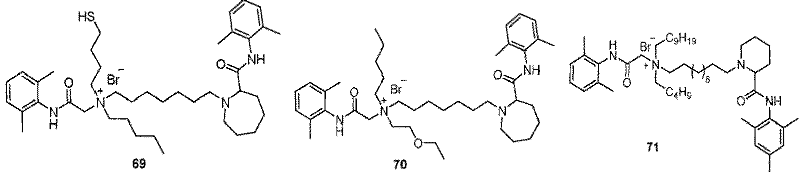

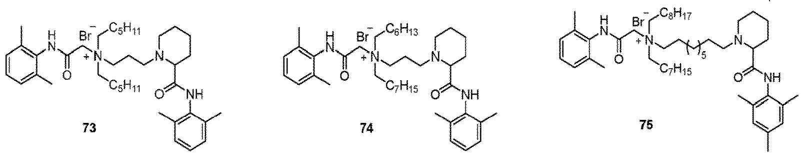

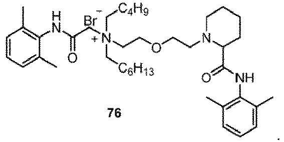

9. Verbindung nach einem der Ansprüche 1 bis 8 oder ein pharmazeutisch unbedenkliches Salz davon oder ein Stereoisomer davon oder ein Solvat davon zur Verwendung als Lokalanästhetikum, wobei die Verbindung zusammen mit einem pharmazeutisch unbedenklichen Träger verwendet wird.

10. Verbindung zur Verwendung nach Anspruch 9 oder ein pharmazeutisch unbedenkliches Salz davon oder ein Stereoisomer davon oder ein Solvat davon, **dadurch gekennzeichnet, dass**
das Lokalanästhetikum die Blockierungszeit sensorischer Nerven länger macht als die Blockierungszeit motorischer Nerven; und/oder
die Lokalanästhesie lange wirkende Lokalanästhesie ist;
vorzugsweise die Anästhesiezeit der Lokalanästhesie über 24 Stunden beträgt.

11. Medikament, **dadurch gekennzeichnet, dass** es sich um eine durch eine Verbindung nach einem der Ansprüche 1 bis 8 oder ein pharmazeutisch unbedenkliches Salz oder ein Stereoisomer davon oder ein Solvat davon unter Zugabe von pharmazeutisch unbedenklichen Hilfsstoffen gebildete Zusammensetzung handelt.

## Revendications

1. Composé de formule 1, ou sel pharmaceutiquement acceptable correspondant, ou stéréoisomère correspondant, ou solvate correspondant : 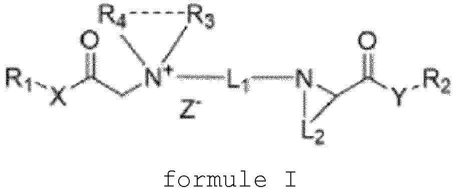
chacun parmi X et Y étant indépendamment choisi dans le groupe constitué par O et NR₁₀, R₁₀ étant choisi dans le groupe constitué par H, deutérium, et C₁-C₄ alkyle ;
Z⁻ étant un anion pharmaceutiquement acceptable ; R₁ étant n₁ aryles substitués par R₁₁ ;
R₂ étant n_{1'} aryles substitués par R_{11'} ;
n₁ et n_{1'} étant chacun indépendamment un entier allant de 0 à 5, et R₁₁ et R₁₁' étant chacun indépendamment choisis dans le groupe constitué par deutérium, C₁-C₄ alkyle, C₁-C₄ alcoxy, halogène, nitro, cyano, hydroxyle, carboxyle, et amino ;
lorsque la ligne en pointillés entre R₃ et R₄ dans la formule 1 est absente, R₃ est un C₁-C₁₀ alkyle substitué ou C₅-C₁₀ alkyle non substitué, et R₄ est indépendamment un C₁-C₁₀ alkyle substitué ou non substitué, ledit substituant étant choisi dans le groupe constitué par deutérium, C₁-C₄ alcoxy substitué ou non substitué, halogène, nitro, cyano, hydroxyle, carboxy, amino, ester, C₁-C₆ alkylthio, et mercapto ; le substituant dudit alcoxy étant un hydroxyle ;
lorsque la ligne en pointillés entre R₃ et R₄ dans la formule I est une liaison, R₃ et R₄ sont indépendamment des C₁-C₄ alkylènes substitués ou non substitués, et le substituant est C₁-C₃ alkyle ; la chaîne principale de l'alkylène contenant 0 à 4 hétéroatomes, et l'hétéroatome étant choisi dans le groupe constitué par O, S, et NR₁₂, ledit R₁₂ étant choisi dans le groupe constitué par hydrogène, deutérium, et C₁-C₄ alkyle ;
L₁ étant un C₁-C₁₄ alkylényle substitué ou non substitué ; la chaîne principale de l'alkylène contenant 0 à 4 hétéroatomes, et l'hétéroatome étant choisi dans le groupe constitué par O, S, et NR₁₂, ledit R₁₂ étant choisi dans le groupe constitué par hydrogène, deutérium, C₁-C₄ alkyle, et C₁-C₄ alcoxy ;
le substituant étant choisi dans le groupe constitué par deutérium, C₁-C₄ alkyle, C₁-C₄ alcoxy, et halogène ;
et L₂ étant un C₁-C₈ alkylényle substitué ou non substitué, et le substituant étant choisi dans le groupe constitué par deutérium, C₁-C₄ alkyle, C₁-C₄ alcoxy, et halogène.

2. Composé selon la revendication 1, ou sel pharmaceutiquement acceptable correspondant, ou stéréoisomère correspondant, ou solvate correspondant, **caractérisé en ce que** :
chacun parmi X et Y est indépendamment NH ou NCH₃ ;
Z⁻ est Br⁻ , Cl⁻ ou sulfonate ;
R₁ est n₁ aryles substitués par R₁₁ ;
R₂ est n_{1'} aryles substitués par R_{11'} ;
chacun parmi n₁ et n_{1'} étant indépendamment un entier allant de 0 à 5, et chacun parmi R₁₁ et R₁₁' étant indépendamment choisi dans le groupe constitué par deutérium, C₁-C₄ alkyle, C₁-C₄ alcoxy, halogène, nitro, cyano, et hydroxyle ;
lorsque la ligne en pointillés entre R₃ et R₄ dans la formule I est absente, R₃ est un C₁-C₁₀ alkyle substitué ou C₅-C₁₀ alkyle non substitué, et R₄ est indépendamment un C₁-C₁₀ alkyle substitué ou non substitué, ledit substituant étant choisi dans le groupe constitué par deutérium, C₁-C₄ alcoxy substitué ou non substitué, hydroxyle, carboxy, C₂-C₅ alkylthio, et mercapto ; le substituant dudit alcoxy étant un hydroxyle ;
lorsque la ligne en pointillés entre R₃ et R₄ dans la formule I est une liaison, R₃ et R₄ sont indépendamment des C₁-C₄ alkylényles, la chaîne principale de l'alkylène contenant 0 à 2 hétéroatomes, et l'hétéroatome étant O ;
L₁ étant un C₃-C₁₄ alkylényle ; le substituant étant alkyle, et la chaîne principale de l'alkylène contenant 0 à 2 hétéroatomes, et l'hétéroatome étant choisi dans le groupe constitué par O et S ;
et L₂ étant un C₁-C₆ alkylényle.

3. Composé selon la revendication 1, ou sel pharmaceutiquement acceptable correspondant, ou stéréoisomère correspondant, ou solvate correspondant, **caractérisé en ce que** :
chacun parmi X et Y est indépendamment NH ou NCH₃ ;
Z⁻ étant Br⁻, Cl⁻ ou sulfonate ;
R₁ étant n₁ aryles substitués par R₁₁ ;
R₂ étant n_{1'} aryles substitués par R_{11'} ;
chacun parmi n₁ et n_{1'} étant indépendamment un entier allant de 2 à 3, et R₁₁ et R₁₁' étant indépendamment choisis dans le groupe constitué par méthyle, propyle, méthoxy, hydroxy, nitro, cyano, et halogène ;
lorsque la ligne en pointillés entre R₃ et R₄ dans la formule I est absente, R₃ étant un C₁-C₈ alkyle substitué ou C₅-C₁₀ alkyle non substitué, et R₄ étant indépendamment un C₁-C₁₀ alkyle substitué ou non substitué, et ledit substituant étant choisi dans le groupe constitué par deutérium, C₁-C₃ alcoxy substitué ou non substitué, hydroxyle, carboxy, C₂-C₅ alkylthio, et mercapto ; le substituant dudit alcoxy étant un hydroxyle ;
lorsque la ligne en pointillés entre R₃ et R₄ dans la formule I est une liaison, R₃ et R₄ étant indépendamment des C₂-C₃ alkylényles substitués ou non substitués, le substituant étant C₁ alkyle, et la chaîne principale de l'alkylène contenant 0 à 1 hétéroatome, et l'hétéroatome étant O ;
L₁ étant un C₃-C₁₄ alkylényle ; la chaîne principale de l'alkylène contenant 0 à 2 hétéroatomes, et l'hétéroatome étant choisi dans le groupe constitué par O et S ;
et L₂ étant un C₁-C₆ alkylényle non substitué.

4. Composé selon la revendication 1, ou sel pharmaceutiquement acceptable correspondant, ou stéréoisomère correspondant, ou solvate correspondant, **caractérisé en ce que** :
chacun parmi X et Y est NH ;
Z⁻ est Br⁻ ;
R₁ est n₁ aryles substitués par R₁₁ ;
R₂ est n_{1'} aryles substitués par R_{11'} ;
chacun parmi n₁ et n_{1'} étant indépendamment un entier allant de 2 à 3, et R₁₁ et R₁₁' étant chacun un groupe méthyle ;
lorsque la ligne en pointillés entre R₃ et R₄ dans la formule I est absente, R₃ étant un C₁-C₈ alkyle substitué ou C₅-C₁₀ alkyle non substitué ; ledit substituant étant choisi dans le groupe constitué par deutérium, C₁-C₃ alcoxy substitué ou non substitué, hydroxyle, carboxy, C₂-C₃ alkylthio, et mercapto ; le substituant dudit alcoxy étant un hydroxyle ;
lorsque la ligne en pointillés entre R₃ et R₄ dans la formule I est une liaison, R₃ et R₄ étant indépendamment un C₂-C₃ alkylényle non substitué ;
la chaîne principale de l'alkylène contenant un hétéroatome, et l'hétéroatome étant O ;
L₁ étant un C₃-C₁₄ alkylényle non substitué ; la chaîne principale de l'alkylène contenant un hétéroatome, et l'hétéroatome étant choisi dans le groupe constitué par O et S ;
et L₂ étant un C₂-C₆ alkylényle non substitué.

5. Composé selon la revendication 1, ou sel pharmaceutiquement acceptable correspondant, ou stéréoisomère correspondant, ou solvate correspondant, **caractérisé en ce que** ledit composé est tel que représenté dans la formule II : 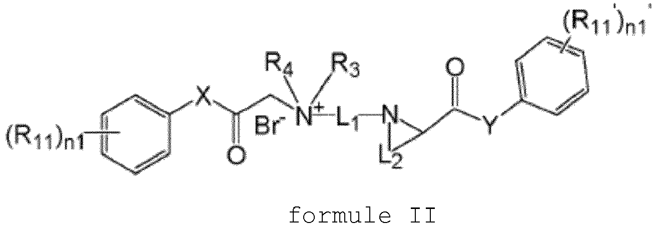
chacun parmi X et Y étant indépendamment NH ou NCH₃ ; chacun parmi n₁ et n₂, étant indépendamment un entier allant de 2 à 3, et R₁₁ et R₁₁' étant choisis dans le groupe constitué par méthyle, propyle, méthoxy, hydroxy, nitro, cyano, et halogène ;
R₃ étant un C₁-C₈ alkyle substitué ou C₅-C₁₀ alkyle non substitué ; R₄ étant indépendamment un C₁-C₁₀ alkyle substitué ou non substitué ; le substituant dudit alkyle étant choisi dans le groupe constitué par deutérium, C₁-C₃ alcoxy substitué ou non substitué, hydroxyle, carboxy, C₂-C₅ alkylthio, et mercapto ; le substituant dudit alcoxy étant un hydroxyle ;
L₁ étant un C₃-C₁₄ alkylényle non substitué ; la chaîne principale de l'alkylène contenant 0, 1 ou 2 hétéroatomes, et l'hétéroatome étant choisi dans le groupe constitué par O et S ;
L₂ étant un C₂-C₆ alkylényle non substitué.

6. Composé selon la revendication 5, ou sel pharmaceutiquement acceptable correspondant, ou stéréoisomère correspondant, ou solvate correspondant, **caractérisé en ce que** ledit composé est tel que représenté dans la formule III : 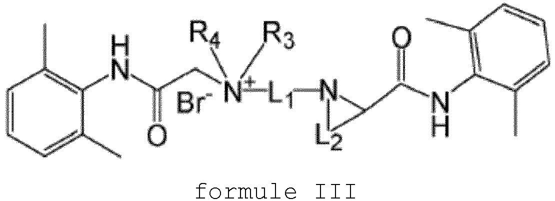
R₃ étant un C₁-C₅ alkyle substitué ; R₄ étant indépendamment un C₁-C₅ alkyle substitué ou non substitué ; le substituant dudit alkyle étant un hydroxyle ;
L₁ étant un C₃-C₆ alkylényle non substitué ;
L₂ étant un C₄ alkylényle non substitué.

7. Composé selon la revendication 1, ou sel pharmaceutiquement acceptable correspondant, ou stéréoisomère correspondant, ou solvate correspondant, **caractérisé en ce que** ledit composé est tel que représenté dans la formule IV : 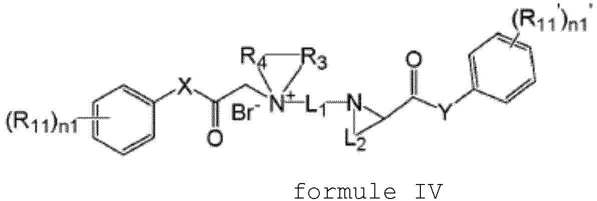
chacun parmi X et Y étant NH ;
chacun parmi n₁ et n₁, étant indépendamment un entier allant de 2 à 3 ; R₁₁ et R₁₁' étant chacun un groupe méthyle ;
R₃ et R₄ étant indépendamment des C₂-C₃ alkylényles substitués ou non substitués ; ledit substituant étant C₁ alkyle, et la chaîne principale de l'alkylène contenant un hétéroatome, et l'hétéroatome étant O ;
L₁ étant un C₃-C₁₄ alkylényle non substitué ; la chaîne principale de l'alkylène contenant 0 ou 1 hétéroatome, et l'hétéroatome étant choisi dans le groupe constitué par O et S ;
L₂ étant un C₂-C₆ alkylényle non substitué.

8. Composé selon l'une quelconque des revendications 1 1 à 7, ou sel pharmaceutiquement acceptable correspondant, ou stéréoisomère correspondant, ou solvate correspondant, **caractérisé en ce que** la structure du composé est l'une des suivantes :

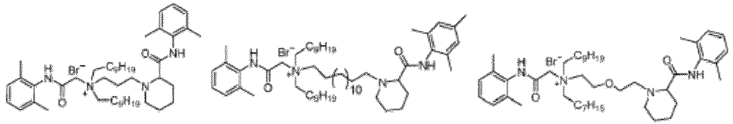

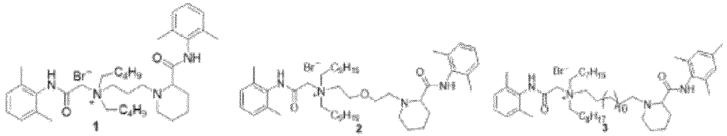

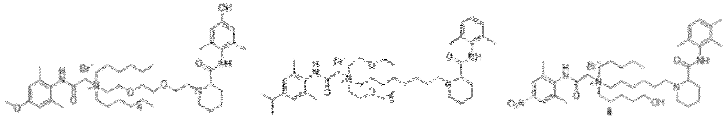

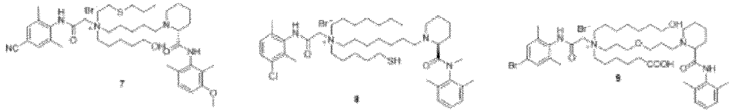

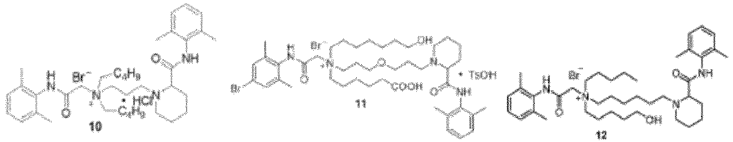

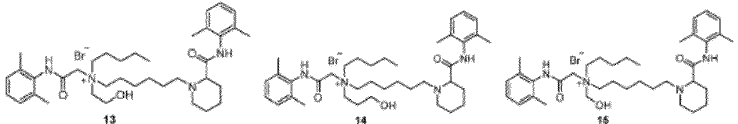

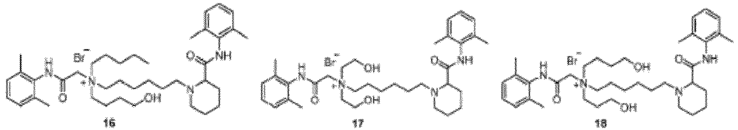

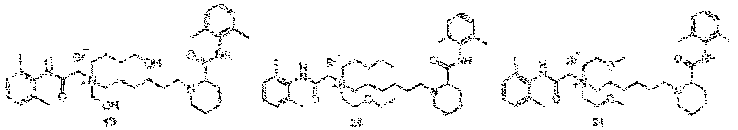

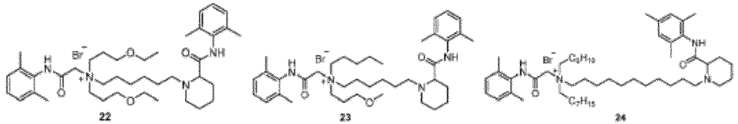

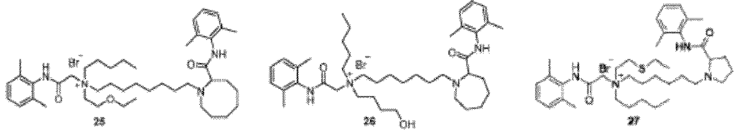

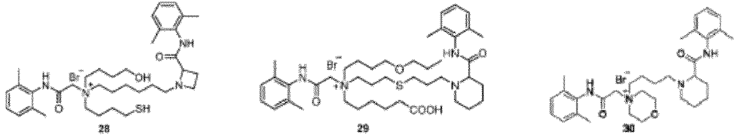

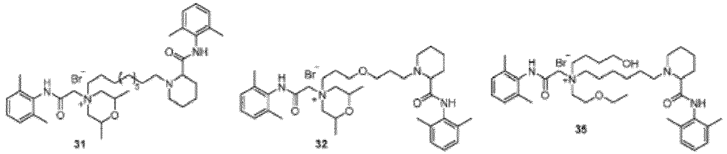

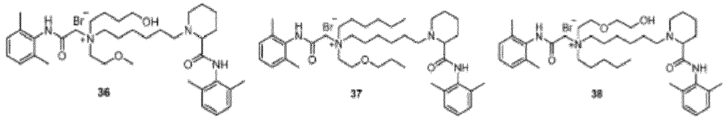

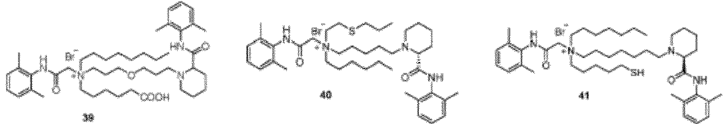

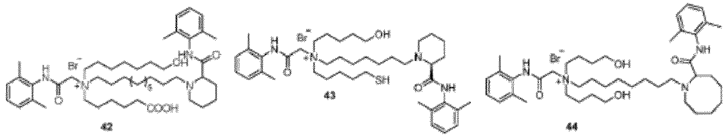

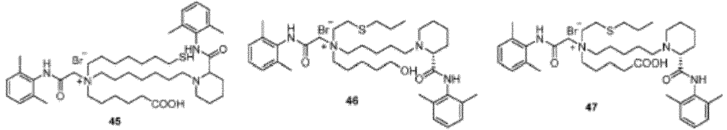

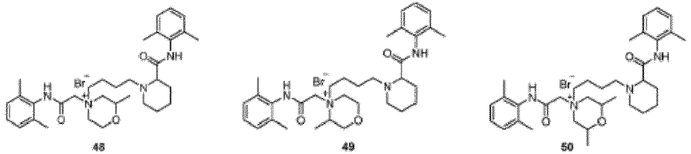

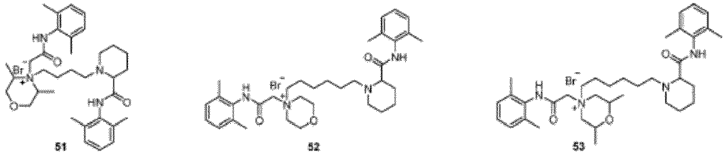

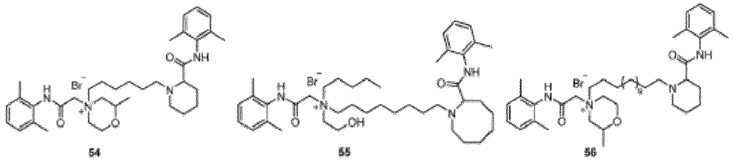

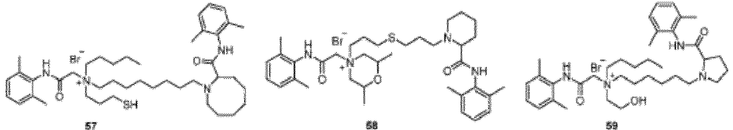

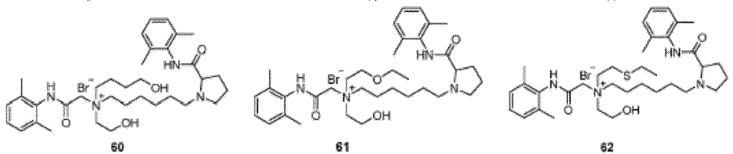

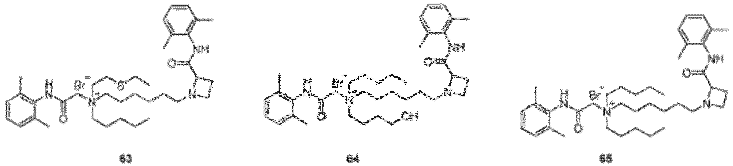

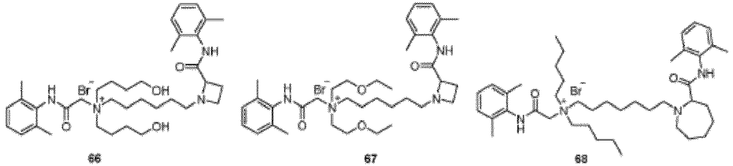

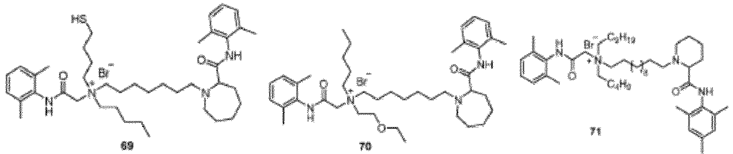

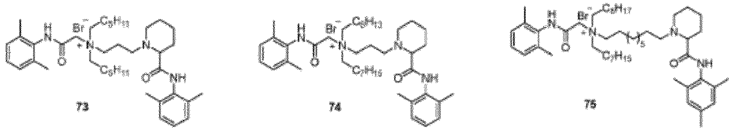

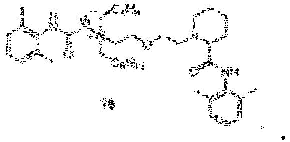

9. Composé selon l'une quelconque des revendications 1 1 à 8, ou sel pharmaceutiquement acceptable correspondant, ou stéréoisomère correspondant, ou solvate correspondant, pour une utilisation comme médicament anesthésique local, ledit composé étant utilisé conjointement avec un support pharmaceutique acceptable.

10. Composé pour une utilisation selon la revendication 9, ou sel pharmaceutiquement acceptable correspondant, ou stéréoisomère correspondant, ou solvate correspondant, **caractérisé en ce que** ledit médicament anesthésique local rend la durée de blocage du nerf sensoriel plus longue que celle du nerf moteur ; et/ou
ladite anesthésie locale étant une anesthésie locale à action longue ;
préférablement la durée d'anesthésie de ladite anesthésie locale dépassant 24 heures.

11. Médicament, **caractérisé en ce qu'**il est une composition formée par un composé selon l'une quelconque des revendications 1 à 8, ou un sel pharmaceutiquement acceptable correspondant, ou un stéréoisomère correspondant, ou un solvate correspondant, avec l'ajout d'excipients pharmaceutiquement acceptables.
